# EUROPÄISCHE PATENTSCHRIFT

(11) **EP 2 410 970 B1**
(45) Veröffentlichungstag und Bekanntmachung des Hinweises auf die Patenterteilung: **13.02.2013**
(21) Anmeldenummer: 10711203.9
(22) Anmeldetag: 24.03.2010
(51) Int. Cl.: A61J 3/07

(54) **VERFAHREN UND VORRICHTUNG ZUR HERSTELLUNG VON WEICHKAPSELN**
NEW DEVICE FOR MANUFACTURING SOFT CAPSULES
DISPOSITIF DE TYPE NOUVEAU DESTINÉ À LA FABRICATION DE CAPSULES SOUPLES

(30) Priorität: 26.03.2009 EP 09156266
(43) Veröffentlichungstag der Anmeldung: 01.02.2012
(73) Patentinhaber: Swiss Caps Rechte und Lizenzen AG, 9533 Kirchberg (CH)
(72) Erfinder: BROCKER, Erich, CH-9533 Kirchberg (CH); PETER, Alois, CH-9500 Wil (CH); SYDOW, Georg, 78467 Konstanz (DE)
(74) Vertreter: Hepp Wenger Ryffel AG
(86) Internationale Anmeldenummer: PCT/EP2010/053824
(87) Internationale Veröffentlichungsnummer: WO 2010/108948

(56) Entgegenhaltungen:
- EP-A- 1 216 680
- EP-A2- 0 227 060
- WO-A-00/28942
- WO-A1-95/17153

## Beschreibung

Die Erfindung betrifft ein Verfahren zur Herstellung von Weichkapseln nach dem Oberbegriff von Anspruch 1. Die Erfindung betrifft ausserdem eine Vorrichtung zur Herstellung von Weichkapseln nach dem Oberbegriff von Anspruch 9.

Weichkapseln werden heutzutage üblicherweise nach dem Rotary-Die-Verfahren hergestellt, welches in der US-2 288 327 erstmals beschrieben wurde. Bei diesem Verfahren werden zwei Filmbänder aus beispielsweise wässrige Gelatine-Lösung über gegenläufig rotierende Formwalzen zusammengeführt und miteinander verschweisst. Diese Formwalzen weisen auf ihrer Oberfläche Mulden (Vertiefungen) auf, welche von Stegen umrandet sind. Die beiden Bänder werden geeignet bis unter den Schmelzpunkt erwärmt und durch Kräfteeinwirkung der Stege miteinander verschweisst. Gleichzeitig werden durch Abquetschen Formkörper in Form von Weichkapseln von den Bändern herausgetrennt. Während des Verschweissens der Bänder wird in die sich dabei bildende Kapsel durch feine Kanäle im Füllkeil Füllgut dosiert. Das Prinzip der Rotary-Die-Verkapselung ist beispielsweise in Fahrig/Hofer, Die Kapsel, Stuttgart 1983, S. 70 ff. beschrieben. Eine Verfahren und eine Vorrichtung für die Herstellung von Weichkapseln unter Verwendung von stärkehaltigen, thermoplastisch verarbeiteten Filmbändern ist beispielsweise in der EP 1 103 254 beschrieben.

Die Dosierung des Füllgutes erfolgt mit Hilfe von Präzisionsdosierpumpen (Kolbedosierpumpen), welche gattungsmässig zu den Hubverdrängungsmaschinen gehören. Das abgemessene Fluid-Volumen wird dabei in mehreren Schüben, abhängig von der Grösse des Volumens über je Kapsel individuelle Leitungen von 30 bis 70cm Länge durch den Füllkeil beidseitig an die Kapselhälften herangeführt. Dabei ist sowohl das Prinzip der hälftigen Dosierung in je beide Kapselhälften (Y-förmiger Duktus) als auch die einkanalige Führung (I-förmiger Duktus) bis an die Spitze des Füllkeils (Kopflochsegment) gebräuchlich. Die entstehenden Kapseln werden dabei gerade soweit aufgewölbt, wie ein Pumpenstoss Füllgut in die Kapseltaschen drückt. Obwohl das Prinzip bereits 1935 beschrieben wurde, ist die Konstruktionsart der Pumpen und des Füllkeils bis zum heutigen Tage im Wesentlichen unverändert geblieben und nach wie vor im Einsatz.

Ein mit dem Rotary-Die-Prinzip nicht vergleichbares Verfahren zum Herstellen umhüllter Tabletten ist in der US 5 8 97 910 beschrieben. Dabei werden zwei Filmbänder zwischen zwei Kalanderwalzen eingeführt, wobei das zu umhüllende Tablettenmaterial in flüssiger Form zwischen die beiden Filme gegeben wird.

In der EP 2 042 165 wurde ein Verfahren beschrieben, mit welchem Schmelzmassen als Füllgut unter Verwendung von thermoplastisch verarbeitetem Hüllenmaterial verkapselt werden können.

In der Regel ist es nach diesem Stand der Technik jedoch nicht möglich, Füllgüter mit einer Viskosität von mehr als ca. 5000 mPa·s zu verkapseln. Auch in der EP 2 042 165 wird vorzugsweise eine konventionelle Rotary-Die-Vorrichtung eingesetzt, bei welcher die Kolbendosierpumpe, mit der die Dosierung des Füllgutes durchgeführt wird, in nicht unerheblichem Abstand (in der Regel etwa 50 cm) vom eigentlichen Ort der Kapselbefüllung im Füllkeil entfernt angeordnet ist und das dosierte Füllgut über diese Strecke durch feine Kanäle im Füllkeil geführt wird. Hochviskose Füllgüter, deren Viskosität stark temperaturabhängig ist, können auf diese Weise nicht abgefüllt werden, da sie durch die feinen Kanäle nicht befriedigend transportiert werden können beziehungsweise dort erstarren. Auch die Absenkung der Viskosität durch Erhöhung der Füllguttemperatur ist nach dem Stand der Technik nur beschränkt machbar, da die Füllguttemperatur unter der Siegeltemperatur der Hüllenbänder liegen muss, was bei Gelatine-Lösungen etwa bei 40°C, für Stärke/Carrageenan-Lösungen bei ca. 60°C zutrifft.

Bei der Herstellung geschmolzener Füllmassen, welch einen oder mehrere Wirkstoffe enthalten (hier API = active pharmaceutical ingredient, wobei darunter auch beispielsweise kosmetische Stoffe oder Nährstoffe zu verstehen sind) konnte festgestellt werden, dass die konventionelle Aufbereitung einer Schmelze in der Regel auch Stabilitätsprobleme aufwirft. Während die konventionelle Bereitstellung einer API-haltigen Matrix deren Abfüllung in Weichkapseln über einen Zeitraum von mehreren Stunden bei Temperaturen bis 40°C ohne nennenswerte Verluste von API durch thermische, oder thermisch-oxidative Zerstörung möglich ist, kann bei Temperaturen über 100°C eine Bevorratung des Füllgutes nicht mehr ohne unerwünschte Folgen gewährleistet werden. EP1216680 offenbart eine Vorrichtung mit allen technischen Marknecher des Oberbegriffs des Anspruchs 9.

Es ist daher eine Aufgabe der vorliegenden Erfindung, ein Verfahren der eingangs genannten Art zu schaffen, mit welchem Weichkapseln mittels des Rotary-Die-Prinzips mit hochschmelzenden bzw. viskosen Füllgütern beschickt werden können. Ausserdem soll eine dazu geeignete Vorrichtung geschaffen werden, welche kostengünstig herzustellen und leicht zu warten ist. Die hochviskosen Schmelzen sollen dabei möglichst präzise dosiert werden können.

Diese Aufgabe wird in verfahrensmässiger Hinsicht mit einem Verfahren gelöst, dass die Merkmale im Anspruch 1 aufweist. Anders als bei den konventionellen Dosierkolbenpumpen, erzeugt das Fördermittel erfindungsgemäss wenigstens einen kontinuierlichen, vorzugsweise druckschwankungsfreien Füllgutstrom. Dieser wird durch einen getrennt vom Fördermittel, vorzugsweise innerhalb des Füllkeils angeordneten Portionierer in Einzelportionen aufgeteilt, bevor diese den Ort der Kapselfüllung erreichen.

Das Grundprinzip der vorliegenden Erfindung besteht somit darin, dass die Dosierung des Füllgutes nicht wie bisher in räumlicher Trennung vom eigentlichen Ort der Kapselbefüllung im Füllkeil stattfindet, sondern direkt an diesem Ort. Der heikle Transport des hochviskosen Füllgutes durch längere feine Kanäle entfällt somit.

Besonders vorteilhaft wird das Füllgut im Fördermittel zu einer viskosen Schmelze mit einer Temperatur von 51° bis 200°C aufbereitet. Das Fördermittel erfüllt damit eine Doppelfunktion als Pumpe und als Aufschmelzvorrichtung.

Vorteilhaft wird der Förderdruck und die Förderstrecke zwischen dem Fördermittel und dem Ort der Kapselfüllung derart dimensioniert, dass das Füllgut nach dem Verlassen des Fördermittels den Ort der Kapselfüllung innerhalb von weniger als 30 Minuten, vorzugsweise innerhalb von weniger als 10 Minuten und noch bevorzugter innerhalb von weniger als 3 Minuten erreicht. Es wurde überraschend festgestellt, dass dieses Zeitfenster von einigen Minuten durchaus ausreichend ist, um sehr viele API's im Füllgut auch bei Temperaturen von 51 bis 200°C noch ausreichend stabil zu halten.

Es ist weiter besonders vorteilhaft, wenn der Füllgutstrom durch den Portionierer in wenigstens zwei Teilströme aufgeteilt wird, welche zu separaten Abfüllorten führen, wobei die Aufteilung derart phasenverschoben erfolgt, dass die endgültige Kapselfüllung an den Teilströmen nacheinander erreicht wird und jeweils der zweite Teilstrom freigegeben wird, bevor der erste Teilstrom unterbrochen wird. Diese Arbeitsweise ermöglicht es, dass ein druckschwankungsfreier Förderstrom zwischen dem Fördermittel und dem Portionierer aufrechterhalten werden kann.

Vorteilhafte Ergebnisse konnten erzielt werden, wenn die Einzelportion des Füllgutstroms zwischen dem Portionierer und dem Ort der Kapselfüllung über eine Distanz von 0 bis maximal 50mm, vorzugsweise maximal 30mm geführt werden. Bei den vorgenannten hohen Temperaturen reichen diese Distanzen aus, um Ablagerungen an den Kanalwänden oder gar eine Verstopfung dieser Kanäle zu verhindern.

Das Füllgut kann zuerst als Mischung von Matrixmaterial und aktiver Substanz aufgeschmolzen und danach im festen, zerkleinerten Zustand zwischengelagert werden. Anschliessend kann das bereits aufbereitete Füllgut erneut aufgeschmolzen und über das Fördermittel allenfalls auch in sehr kleinen Chargen dem Portionierer zugeführt werden. Damit ist ein gewünschtes Füllgut stets abrufbar, ohne dass die aktive Substanz beschädigt wird.

Die Förderung des Füllgutstroms zum Portionierer erfolgt vorteilhaft mit einem Umlaufverdrängungsfördermittel. Dabei kann es sich um eine Schraubenpumpe und bevorzugt um eine Schmelzextrusionsvorrichtung handeln. Schraubenpumpen haben den Vorteil einer pulsationsfreien Förderung. Denkbar wären aber beispielsweise auch Zahnradpumpen oder Flügelpumpen.

Weitere Vorteile lassen sich mit dem Verfahren erzielen, wenn der Füllgutstrom bei der Dosierung bei Raumtemperatur, also bei einer Temperatur von 0° bis 60°C, vorzugsweise von 0° bis 50°C, eine dynamische Viskosität von mehr als 5000 mPa·s aufweist. Beträgt die Temperatur dagegen mehr als 50°C, sollte die Viskosität mehr als 10 mPa·s, bevorzugter mehr als 1000 mPa·s und noch bevorzugter mehr als 5000 mPa·s betragen.

In vorrichtungsmässiger Hinsicht wird die erfindungsgemäss gestellte Aufgabe mit einer Vorrichtung gelöst, welche die Merkmale im Anspruch 9 aufweist. Es ist dabei besonders bevorzugt, den Portionierer innerhalb des Füllkeils anzuordnen. Innerhalb des Füllkeils bedeutet zwischen den beiden konvexen Keilflächen und möglichst nahe an deren Oberfläche. In bestimmten Fällen und je nach Dimensionierung des Füllkeils wäre es aber auch denkbar, den Portionierer ausserhalb des Füllkeils zu platzieren, also beispielsweise an einer Stirnseite oder an der Oberseite des Füllkeils.

Wie bereits erwähnt ist das Fördermittel vorteilhaft ein Umlaufverdrängungsfördermittel, insbesondere eine Schraubenpumpe und bevorzugt eine Schmelzextrusionsvorrichtung. Zwischen dem Portionierer und dem Ort der Kapselfüllung an den Oberflächen des Füllkeils sind Verteilkanäle angeordnet, deren maximale Länge 50mm, vorzugsweise aber lediglich 30mm beträgt.

Zur Verhinderung von Druckstössen ist der Füllgutstrom am Portionierer in wenigstens zwei Teilströme aufteilbar, welche direkt oder über getrennte Verteilkanäle zu separaten Abfüllorten leitbar sind, wobei die Teilströme phasenverschoben nacheinander erzielbar sind. Zu diesem Zweck sind entsprechend von sich zwei sinusartig überlagernder Kurven Öffnungsquerschnitte für jeden Teilstrom nacheinander freilegbar und wieder verschliessbar.

Der Portionierer kann wenigstens eine Dosierungshohlwelle aufweisen, welche drehbar im Füllkeil gelagert ist und welche Ausgabeöffnungen aufweist, welche in wenigstens einer Drehwinkellage mit einer Öffnung bzw. mit einem Kanal im Füllkeil korrespondiert. Eine derartige Dosierungshohlwelle lässt sich platzsparend und nahe an den Oberflächen des Füllkeils unterbringen. Die Dosierungshohlwelle kann von den beiden Stirnseiten oder von der Oberfläche des Füllkeils her in jeder beliebigen Relativlage in den Füllkeil eindringen. Besonders vorteilhaft ist es jedoch, wenn die Dosierungshohlwelle parallel zu den Drehachsen der Formwalzen gelagert ist und wenn jede Ausgabeöffnung entweder mit einem zu den Keilflächen oder mit einem zur Keilspitze des Füllkeils führenden Kanal verbindbar ist. Auch der Einsatz mehrerer Dosierungshohlwellen beispielsweise auf jeder Längsseite des Füllkeils wäre denkbar. Wenn die Dosierungshohlwellen gegenläufig rotieren, könnte ein durch Reibung verursachtes Drehmoment auf den Füllkeil neutralisiert werden.

Der Portionierer könnte aber auch wenigstens einen Dosierschieber aufweisen, der linear verschiebbar im Füllkeil gelagert ist und mit dem taktweise Zufuhrkanäle für das Füllgut freilegbar bzw. verschliessbar sind. Auch ein derartiger Dosierschieber könnte von jeder Seite des Füllkeils her mit Ausnahme der Keilflächen in den Füllkeil eindringen. Auch hier wäre der Einsatz mehrerer Dosierschieber oder Dosierschieberpaare denkbar. Vorteilhaft wird der wenigstens eine Dosierschieber senkrecht zu einer durch die beiden Drehachsen der Formwalzen verlaufenden Ebene gelagert. Dies hat den Vorteil, dass auf den schwimmend gelagerten Füllkeil kein Drehmoment ausgeübt wird und dass für die Lagerung über dem Füllkeil mehr Platz zur Verfügung steht.

Der Dosierschieber könnte aber auch als Dosierrohr ausgebildet sein, über das Füllgut zuführbar ist. Die Funktion des Dosierrohrs wäre damit vergleichbar mit derjenigen der Dosierungshohlwelle, wobei die Bewegung jedoch nicht rotativ, sondern linear verläuft.

Denkbar wäre schliesslich auch die Ausgestaltung des Portionierers als Ventil mit wenigstens einer Ventilnadel, die mit einem Ventilsitz im Füllkeil zusammenwirkt. Die Unterteilung des Fördergutstroms könnte so je nach Dimensionierung des Ventilsitzes mit sehr geringen Ventilhüben erfolgen.

Gemäss der vorliegenden Erfindung soll unter einem hochviskosem Füllgut ein Material verstanden werden, welches eine Viskosität von mehr als 5000 mPa·s bei der Dosierung in Weichkapseln aufweist. Da in der Regel ein Übergang von fest zu flüssig (Schmelze) bzw. eine Erniedrigung der Viskosität bei Erhöhung der Temperatur erfolgt, ist dieser Grenzwert von 5000 mPa·s bei der für eine Dosierung und Verkapselung technisch niedrigstmöglichen Temperatur zu verstehen.

Gemäss der vorliegenden Erfindung wird das Füllgut in einer Fördereinrichtung viskoser Schmelzen in Form einer Schmelze erzeugt. Anschliessend wird das geschmolzene Füllgut durch einen zumindest teilweise direkt im Füllkeil angeordneten Portionierer geführt. Dieser Portionierer besteht bevorzugt aus einer drehbaren Dosierungswelle, die nachstehend näher beschrieben wird.

Gemäss der vorliegenden Erfindung wird unter einer Fördereinrichtung viskoser Schmelzen vorzugsweise eine Schmelzextrusionsvorrichtung oder eine Schmelzepumpe (1- oder 2-Wellenxtruder) verstanden. Der Portionierer ist an einem Ende direkt mit der Fördereinrichtung viskoser Schmelzen verbunden und beweglich in den Füllkeil eingebaut.

In einer anderen Ausführungsform gemäss der vorliegenden Erfindung kann der Portionierer in Form eines Schiebers ausgebildet sein, wobei der Dosierschieber an einem Ende mit der Schmelzextrusionvorrichtung verbunden ist und an einem, in einem Füllkeil befindlichen Abschnitt mindestens eine Perforation aufweist, welche durch Vor- und Zurückschieben einer Verschlusseinheit geöffnet beziehungsweise geschlossen werden kann. Die nachstehend für eine Dosierungswelle gezeigten Anordnungen sind auch für einen solchen Schieber anwendbar, mit dem Unterschied, dass die Dosierkanäle nicht mit Hilfe einer Drehbewegung, sondern mit Hilfe einer Vor-Rückwärtsbewegung freigegeben werden.

Eine weitere Ausführungsform gemäss der vorliegenden Erfindung kann der Portionierer kann in einem Dosierkanal bestehen, welcher an einem Ende mit der Schmelzextrusionvorrichtung verbunden ist und an einem, in einem Füllkeil befindlichen Abschnitt mindestens eine Perforation aufweist, wobei der Dosierkanal starr im Füllkeil angeordnet ist und die mindestens eine Perforation über ein Ventil geöffnet beziehungsweise geschlossen werden kann. Vorzugsweise können die Ventile über eine Nockenwelle gesteuert werden.

Die nachstehend näher gezeigte Dosierungswelle ist konstruktiv bevorzugt, da sie durch Auswahl der Materialien z.B. mit PTFE (Polytetrafluorethylen) beschichteter Stahl, gehärteter Stahl, oder Keramik exakt eingepasst werden kann und mit oder ohne Schmier- und Dichtungsmittel betrieben werden kann. Ausserdem kann die Dosierungswelle getaktet gedreht/geschwenkt oder kontinuierlich gedreht werden und die Bewegungsgeschwindigkeit kann der Viskosität, Kapselgrösse, den Füllkanaldurchmessern usw. einfach angepasst werden.

Ein Abschnitt der Dosierungswelle befindet sich im Füllkeil und weist Perforationen auf. Diese Perforationen (Löcher) verbinden die Dosierwelle entweder direkt oder über kurze Kanäle mit Mulden in den Formwalzen.

Die Dosierwelle ist so im Füllkeil angeordnet, dass ihr Perforationen aufweisender Abschnitt direkt am eigentlichen Ort der Kapselbefüllung oder nur wenig davon entfernt positioniert ist.

Gemäss einer Ausführungsform der vorliegenden Erfindung ist zwischen Portionierer und Füllgutaustrittsloch ein Dosierkanal mit einer Länge von weniger als 50 mm, bevorzugt weniger als 30 mm vorhanden. Idealerweise ist jedoch der Portionierer, vorzugsweise die Dosierwelle, direkt der Verschliessmechanismus des Füllgutloches, und ein eigentlicher Füllgutkanal entfällt.

Das hochviskose Füllgut wird durch die vergleichsweise dicke Dosierwelle problemlos in den Füllkeil bis zum eigentlichen Ort der Kapselbefüllung geführt. Vorteilhaft ist insbesondere, dass auf dieser kurzen Wegstrecke kaum unkontrollierter Druck- oder Temperaturabfall erfolgen kann.

Eine viskose Masse kann durch die Perforationen in dem Portionierer, vorzugsweise der Dosierwelle, in die Mulden der Formwalzen dosiert werden, wenn die Perforationen und Mulden miteinander kommunizierend verbunden sind. Dies ist in einer ersten Position der Fall, in der entweder die Perforationen der Dosierwelle sich genau in die Mulden der Formwalzen öffnen, oder in der die Perforationen der Dosierwelle passgenau zu Öffnungen von im Füllkeil vorgesehenen Kanälen angeordnet sind, wobei die Ausgangsöffnungen der Kanäle ihrerseits passgenau zu den Mulden in den Formwalzen positioniert sind.

Solange diese kommunikative Verbindung besteht, wird das Füllgut in die Mulden der Formwalzen dosiert. Diese kommunikative Verbindung wird dadurch aufgehoben, dass die Dosierwelle aus der ersten Position gedreht wird, so dass sich die Perforationen der Dosierwelle nicht länger direkt in die Mulden der Formwalze oder in die Verbindungskanäle im Füllkeil öffnen. Durch Drehung der Dosierwelle mit einer bestimmten Geschwindigkeit kann somit erreicht werden, dass eine genaue dosierte Menge an Füllgut durch die Perforationen in die Mulden der Formwalzen gelangt.

Damit wird gemäss der vorliegenden Erfindung ein völlig anderes Dosierprinzip angewandt als bisher im Stand der Technik mit Dosierkolbenpumpen üblich war. Die Dosis ist nicht mehr ein vorher durch Kolbenvolumen abgemessenes und durch mehrfach hintereinander geschaltes Fördern einer hydrostatischen "Säule", sondern ein durch definierte Öffnungen in Abhängigkeit der Zeit, der Temperatur, der Viskosität und des Druckes in die Kapsel austretendes Volumen.

Eine erfindungsgemässe Dosierwelle hat bevorzugt mindestens die Länge eines Füllgutkeiles, also 7.5 inches, 10 inches usw., einen Durchmesser von 2-5 cm und eine Wanddicke von 1-5 mm. Die Bohrungen (Perforationen) für den Austritt des viskosen Füllgutes können rund oder schlitzartig ausgeprägt sein.

Die Dosierung kann auch durch entsprechende Drehung der Formwalzen erreicht werden. Es ist aber bevorzugt, die Abscherung des Füllgutstroms durch Drehung der Dosierwelle zu erreichen, da dann diese Aufgabe nicht durch die empfindlichen Filmbänder übernommen werden muss beziehungsweise kein Füllgut zwischen Füllkeil und Band treten kann.

Sehr ähnlich kann ein Dosierschieber, also ein stabförmig rundes oder quaderförmig flaches oder komplexer geformtes, hohles mit Füllgut gefülltes Formteil, verwendet werden, das ebenfalls Austrittsöffnungen hat, deren Öffnungs- und Verschliessmechanismus aber nicht durch Drehen oder Wippen, sondern durch Vor- und Zurückschieben funktioniert.

Ebenso können in einem starren Dosierkanal (z.B. Dosierzylinder) angeordnete Perforationen (Löcher) durch Ventile für den Durchlass von viskosem Füllgut geöffnet und verschlossen werden.

Gemäss einer bevorzugten Ausführungsform der vorliegenden Erfindung weist die Dosierungswelle an ihrem im Füllkeil befindlichen Abschnitt mindestens zwei Perforationen auf, welche in einer ersten Position direkt in Mulden von zwei Formwalzen führen und in einer zweiten Position von den Mulden in den Formwalzen getrennt sind, wobei die erste Position durch Drehung entweder der Dosierungswelle oder der Formwalzen in die zweite Position überführt werden kann. Bevorzugt ist die erste Variante (Drehung der Dosierwelle), da dann wie vorstehend geschildert die Abscherung des Füllgutstroms durch Drehung der Dosierwelle erreicht wird.

Gemäss weiteren Ausführungsformen der vorliegenden Erfindung ist im Füllkeil mindestens ein Kanal vorhanden, welcher in einer ersten Position eine Perforation der Dosierungswelle mit Mulden der Formwalzen kommunizierbar verbindet. Dieser Kanal ist im Vergleich zu den im Stand der Technik vorhandenen Kanälen im Füllkeil kurz. Während im Stand der Technik wie beschrieben das Füllgut in einer entfernten Dosierpumpe dosiert und anschliessend durch feine Kanäle von 1-3 mm Durchmesser über eine Strecke von etwa 50 cm zum eigentlichen Befüllungsort geführt werden muss, dienen die Kanäle bei der vorliegenden Erfindung nur zur Überbrückung der kurzen Distanz zwischen den Perforationen der innerhalb des Füllkeils angeordneten Dosierwelle zu den Mulden in der Formwalze. Innerhalb dieser kurzen Distanz kommt es auch bei der Verwendung von hochviskosem Füllgut zu keinen Problemen wie Verfestigung oder Verstopfung der Kanäle.

Bevorzugt sind die Kanäle nicht länger als 30 mm, noch bevorzugter nicht länger als 5 mm. Der Durchmesser des Füllkanals beträgt mindestens 1 mm, bevorzugter 2 mm, noch bevorzugter 1/3 des Kapseldurchmessers, bzw. 1/3 der am Füllloch vorbeilaufenden Napfbreite.

Gemäss der vorliegenden Ausführungsform sind verschiedene Ausgestaltungen des oder der Kanäle möglich. Beispielweise kann ein einziger Kanal vorhanden sein, der in einer ersten Position eine Perforation der Dosierwelle mit einem aus zwei Mulden verschiedener Formwalzen gebildeten Hohlraum verbindet und Füllgut in diesen Hohlraum führen kann (Kopflochbefüllung).

Gemäss einer anderen Ausführungsform ist im Füllkeil ein Kanal vorhanden, der Y-förmig ausgestaltet ist. Mit anderen Worten teilt sich der Kanal ausgehend von dem zur Perforation weisenden Ende in der Dosierwelle in zwei Kanäle auf, welche zu jeweils einer Mulde einer Formwalze führen. Hierbei werden die Mulden der Formwalzen separat befüllt, so dass sich bei der anschliessenden Verschweissung zur finalen Kapsel das Füllgut bereits im Kapselinneren befindet.

Gemäss einer weiteren Ausführungsform sind im Füllkeil mehrere separate Kanäle vorgesehen, die sequentiell mit Perforationen der Dosierwelle und Mulden in Formwalzen verbunden werden können. Auf diese Weise wird eine getaktete Dosierung des Füllguts möglich. Vorzugsweise sind hierbei vier separate Kanäle im Füllkeil vorgesehen, welche sich paarweise links und rechts der Dosierwelle befinden, so dass in einer ersten Position die beiden im Füllkeil weiter unten angeordneten Kanäle zwei Perforationen der Dosierungswelle kommunizierend mit Mulden der Formwalzen verbinden, und in einer zweiten Position die beiden im Füllkeil weiter oben angeordneten Kanäle zwei Perforationen der Dosierungswelle kommunizierend mit Mulden der Formwalzen verbinden, wobei die erste Position durch Drehung der Dosierungswelle in die zweite Position überführt werden kann.

Die Anzahl an Perforationen in der Dosierwelle kann den Prozessbedingungen entsprechend vom Fachmann routinemässig gewählt werden. In einer üblichen erfindungsgemässen Ausgestaltung weist die Dosierwelle vier Perforationen auf. Diese Perforationen sind vorzugsweise jeweils um ein Viertelkreissegment voneinander beabstandet. Bei der vorstehend beschriebenen Ausführungsform mit mehreren, vorzugsweise vier separaten Kanälen kann es aber vorteilhaft sein, einen geringeren Abstand zwischen je zwei benachbarten Perforationen zu wählen, beispielsweise ein Achtelkreissegment. Entsprechend vergrössert sich dann der Abstand zur nächsten Perforation (im Fall von 4 Perforationen).

Die Perforationen sind am im Füllkeil befindlichen Abschnitt der Dosierwelle derart angeordnet, dass sie mit den Mulden der Formwalzen direkt oder über Kanäle im Füllkeil kommunizierend verbunden werden können, und weisen vorzugsweise einen Durchmesser auf, welcher dem Durchmesser des oder der Kanäle im Füllkeil entspricht.

Die Dosierwelle kann in einen üblicherweise verwendeten Füllkeil einer Rotary-Die-Vorrichtung eingebaut werden. Hierzu ist eine Bohrung in dem Füllkeil zu erzeugen, welche einen leicht grösseren Durchmesser als der Aussendurchmesser der Dosierwelle aufweist, so dass die Dosierwelle innerhalb des Füllkeils reibungslos gedreht werden kann. Gegenüber einem herkömmlichen Füllkeil ist des Weiteren der sonst übliche lange Kanal von der (bei der erfindungsgemäss normalerweise nicht vorhandenen) Kolbendosierpumpe zum eigentlichen Befüllungsort nicht erforderlich und deshalb in der Regel wegzulassen. Stattdessen ist der vorstehend beschriebene mindestens eine Kanal im Füllkeil vorzusehen, der mindestens eine Perforation der Dosierwelle mit Mulden der Formwalzen verbindet.

Gemäss einer Ausführungsform der vorliegenden Erfindung kann jedoch zusätzlich zu der hier beschriebenen Dosiervorrichtung eine herkömmliche Füllgut-Zuführung mittels separater Kolbendosierpumpe vorhanden sein, damit die erfindungsgemässe Rotary-Die-Vorrichtung bei Bedarf auch auf herkömmliche Weise betrieben werden kann.

Die Dosierwelle wird erfindungsgemäss durch den Füllkeil hindurchgeführt. Das eine Ende der Dosierwelle ist wie vorstehend beschrieben mit einer Fördereinrichtung viskoser Schmelzen verbunden. Das andere Ende der Dosierwelle, welches den Füllkeil auf der von der Fördereinrichtung viskoser Schmelzen abgewandten Seite verlässt, ist mit einem Motor verbunden, welcher die Dosierwelle in eine Drehbewegung versetzen kann. Hierbei kann jeder übliche Motor verwendet werden, welcher der Dosierwelle eine kontinuierliche oder oszillierende Bewegung auflegen kann.

Die Drehgeschwindigkeit oder Oszillationsgeschwindigkeit der Dosierwelle kann vom Fachmann entsprechend gewünschten Prozessbedingungen frei gewählt werden und wird so auf die Geschwindigkeit der Andienung der Kapselnäpfe synchronisiert, dass eine Befüllung kurz nach Positionierung des Füllloches innerhalb der Umrandung des Kapselnapfes beginnt und kurz vor Positionierung des Füllloches vor der gegenüberliegenden Umrandung des Kapselnapfes aufhört.

Der vorstehend beschriebene Füllkeil mit Dosierwelle, oder der Dosierschieber, oder der Dosierkanal mit Ventilen, wird erfindungsgemäss mitsamt der Schmelzextrusionvorrichtung in eine herkömmliche Rotary-Die-Vorrichtung eingebaut. Rotary-Die-Vorrichtungen und das Rotary-Die-Verfahren sind allgemein bekannt und beispielsweise in der EP-1 103 254 A1 beschrieben. Auf die entsprechende Offenbarung der EP-1 103 254 A1 wird ausdrücklich Bezug genommen.

Für die Zubereitung des Füllgutes kann jede herkömmliche Schmelzextrusionvorrichtung verwendet werden. Diese sind dem Fachmann bekannt und müssen hier nicht im Detail erläutert werden.

Um etwaige, durch den Betrieb der Fördereinrichtung viskoser Schmelzen bedingte Druckschwankungen in der Dosierwelle auszugleichen, kann gemäss einer bevorzugten Ausführungsform der Erfindung eine Pumpe mit einer Differentialdruckreguliereinheit an der Dosierwelle bereitgestellt sein, beispielsweise zwischen der Fördereinrichtung viskoser Schmelzen und der Dosierwelle.

Mit der vorliegenden Erfindung gelingt es, Weichkapseln mit schmelzbarem Füllgut bei höchstmöglicher thermischer Schonung mittels Rotary-Die-Technik herzustellen und die Technologie der Dosierung vor allem bei hohen Viskositäten des Füllgutes zu verbessern.

Um diesen Effekt für die Herstellung von Kapseln mit hochschmelzenden Füllgütern auszunützen, sind einige Grundsätze zu befolgen und mit der erfindungsgemässen Vorrichtung umzusetzen:
o Zeitverzugsfreie kontinuierliche Herstellung des Füllgutes und Befüllung der Weichkapseln (kein Batchbetrieb);
o Dosieren von API, Mischen mit der Füllgutmatrix und Aufschmelzen zu einer homogenen Masse oder Wiederaufschmelzen einer erkalteten homogenen Schmelze unmittelbar vor Dosierung und Verkapselung ("Melt-On-Demand");
o Druckschwankungsarmes genaues Dosieren;
o Sanftes Abscheren der portionierten Dosis vom hochviskosen "Strang" ohne Beteiligung der /ohne Berührung mit der Hüllenmasse;
o Kontrolle /Kompensation von Wärmeverlusten;
o Schnelle Auskühlung nach Formung;

Die vorliegende Erfindung betrifft somit auch ein Verfahren zur Herstellung von Weichkapseln, umfassend die Schritte
a) Zubereitung eines Füllgutes in einer Fördereinrichtung viskoser Schmelzen bei einer Temperatur im Bereich von 51°C bis 200°C,
b) Kontinuierliche Zuführung des Füllgutes in das Innere einer durch zwei Filmbänder mittels von Formwalzen gebildeten Kapsel,
dadurch gekennzeichnet, dass die Schritte a) und b) in einer vorstehend beschriebenen Vorrichtung innerhalb eines Zeitraums von weniger als 30 Minuten, vorzugsweise weniger als 10 Minuten und noch bevorzugter weniger als 3 Minuten durchgeführt werden.

Mit der Vorrichtung gemäss der vorliegenden Erfindung können diese Grundsätze gleichzeitig befolgt werden. Mit dem vorstehend beschriebenen, für das Dosieren mit hohen Viskositäten ausgelegten Füllkeil wird eine direkte Ankopplung von Zuführungen von Füllmasse aus Schmelzextrusionsgeräten unter erhöhtem Druck und erhöhter Temperatur erlaubt. Es kommt somit bei der erfindungsgemässen Vorrichtung zu einer minimalen Auskühlung unter Erhaltung eines gleichmässigen Druckes. Das Füllgut wird örtlich und zeitnah angedient.

Gemäss der vorliegenden Erfindung erfolgt das Einbringen des Füllgutes im Kapselformungsschritt (z.B. der Rotary-Die-Methode) ebenfalls bei erhöhter Temperatur, die mindestens so hoch liegt, das das geschmolzene Füllgut ausreichend fliesst und dosiert werden kann, aber um 10-150°C niedriger, bevorzugt 20-50°C niedriger als die Siegel-Verschweisstemperatur der thermoplastischen Hüllfilme.

Es hat sich gezeigt, dass der konventionelle Ansatz von Füllgut im Batchbetrieb, also die Herstellung einer grösseren Menge Füllgut in einem Ansatz, bevorzugt in einem Tank, eine erhebliche thermische Belastung der darin enthaltenen API bedeutet, insbesondere da die Abfüllung einer Masse in der Regel nach der Herstellung mehrere Stunden bis Tage beansprucht, und das gesamte Füllgut auf erhöhter Temperatur in fliessfähigem Zustand gehalten werden muss.

Die Stabilität wird in der Regel zunehmend beeinträchtig durch Zeit, Temperatur (Arrhenius-Gleichung), der Kristallinität (bzw. molekulardisperser Verteilung (Lösung)) und Vorliegen anderer reaktiver Stoffe (Stoffgemische).

Die Herstellung solcher Massen und die Problematik des thermischen Abbaus wurden bereits publiziert. Eine Abfüllung in Kern-Hülle-Formkörper wie Weichkapseln ist aber neu. Überraschenderweise hat sich gezeigt, dass für die meisten gebräuchlichen pharmazeutischen, "nutritional" Wirkstoffe (Vitamine, Mineralstoffe etc) (hier generell API genannt) das Temperatur-Zeitprofil, welches sich aus der Verarbeitung in einem Extruder und entsprechend schneller Dosierung in Rotary-Die-Verkapselung bei höheren Temperaturen ergibt, ohne negative Einwirkungen bleibt. Dies insbesondere, wenn
a) die einzelnen Rohstoffkomponenten unter Vakuum bzw. Schutzgas aufgeschmolzen wurden;
b) eine Mischung der Komponenten im letzten Moment vor der Einbringung in die Kapsel erfolgt (insbesondere geeignet ist eine kontinuierliches Aufschmelz-, Misch- und Entgasungsverfahren wie in einem Ein- oder Zweiwellenextruder);
c) die Dosierung in die Kapselhälften in kürzester Zeit nach Aufschmelzung erfolgt, welches erfindungsgemäss durch eine neue Art der Masseführung und Dosierung erfolgt;
d) eine schnelle Abkühlung der hergestellten Kapsel erfolgt;
e) das thermoplastische Hüllenmaterial tolerant gegenüber Erweichungs- und Schockkühlungszyklen ist;
f) Wasserdampfdruck bzw. Wasserkonzentration bei den Vorgängen Verkapselung und Kühlung beachtet wird (wasserarm).

Obwohl die vorliegende Erfindung auf den Einsatz von hochviskosen Füllgütern ausgelegt ist, ist es mit der erfindungsgemässen Vorrichtung auch sehr gut möglich, niedrigviskose Füllgüter befriedigend füllen zu können, da isobar gefördert und dosiert wird, kein hydrostatisches Volumen mitschwingt und direkt an der Kapsel ohne Totvolumen dosiert wird.

Erfindungsgemäss bevorzugt wird das Füllgut unmittelbar vor der Verkapselung kontinuierlich aus den entsprechenden Ausgangskomponenten durch Schmelzen und Mischen hergestellt.Vorzugsweise wird mindestens ein Wirkstoff in fester Form kontinuierlich zusammen mit einer geeigneten Füllgut-Matrix in einen 2-Wellenextruder dosiert, gemischt und zusammen aufgeschmolzen und idealerweise - abhängig von den gewählten Komponenten - gelöst. Es entsteht eine molekulardisperse Schmelze.

Unter "Molekulardispers" wird gemäss der vorliegenden Erfindung eine Lösung oder lösungsähnliche bis kolloidale Verteilung des API verstanden, welche im wesentlichen frei von makroskopisch feststellbaren Konzentrationshäufungen des API in kristalliner oder amorpher Form ist. Die molekulardisperse Phase kann flüssig (Lösung), halbfest (fest und flüssig gemeinsam vorliegend) oder fest (Glas oder teilkristallin/kristallin) sein.

Die Länge (L) und der Durchmesser (D) sowie die Konfiguration der innenliegenden Förderschnecken der Fördereinrichtung viskoser Schmelzen kann vom Fachmann entsprechend den gewünschten Anwendungsbedingungen routinemässig ausgewählt werden.

Das Gleiche gilt für das Temperaturprofil, den Druck an der Austrittsdüse, die Verweilzeit und die Förderleistung im Extruder. Allerdings sind diese Variablen nicht ganz unabhängig voneinander, so dass es sich als vorteilhaft erweisen kann, über eine Schmelzepumpe zum Beispiel Fördervolumen und Druck für die Andienung in das Dosiersystem verschieden vom Druck des Extruders zu wählen.

In einer weiteren erfindungsgemässen Variante kann die Schmelzematrix allein oder als API-haltige Schmelze zu einem Strang geformt, gekühlt, geschnitten und zwischengelagert werden.

Logistisch besonders vorteilhaft kann in einem weiteren Schritt in einem sogenannten "Melt-On-Demand-System", wie es kommerziell z.B. von Robatech, Muri, Schweiz oder ITW Dynatec, 31 Volunteer Drive, Hendersonville, TN erhältlich ist, das Material portionsweise schnell, kontrolliert und schonend wieder aufgeschmolzen, und über eine Schmelzepumpe mit den für das Kapseldosiersystem gewünschten Drücken, Temperaturen und Förderleistungen wieder angedient werden.

In einer weiteren Ausführungsform kann der API in kristalliner Form in eine bereits geschmolzene Matrix eingetragen werden, wenn ein weiterer Dosierer den API im Verlauf der 2-Wellenextrusion einmischt, oder wenn die in einem ersten Extruder hergestellte Matrix-Mischung in einen weiteren Extruder eingeknetet wird. Der Fachmann kann bei dieser Ausführungsform (1-Welle, 2-Welle) die Grösse und Länge (L/D) der Extruder entsprechend den gewünschten Anwendungsbedingungen routinemässig (Stoff, Fluss, Temperatur, Viskosität, Zeit) wählen. So kann - wenn gewünscht- auch ein Erhalt der Kristallinität des API sichergestellt werden. Besonders bevorzugt wird der aktive Stoff in kontrollierter Korngrösse oder mikropartikulärer Form (Mikronisiert, pelletisiert oder gecoated) der Schmelze zugesetzt.

In einer Ausführungsform für schnelle Schmelz- und Mischvorgänge und niedrigviskosen Massen kann nach der Herstellung in Extrudern neben der erfindungsgemässen Dosierung zusätzlich direkt über eine konventionelle Kolbenpumpe in eine Weichkapsel mit thermoplastischer Hülle dosiert werden.

In einer besonders bevorzugten Ausführungsform wird einem oder mehreren Extrudern oder Melt-On-Demand-Systemen eine Schmelzepumpe mit Differenzialdruck-Controller nachgeschaltet. Diese ermöglich eine praktisch druckschwankungsfreie Förderung der Schmelze in den Dosierer. Solche Systeme sind kommerziell erhältlich, z.B. bei Harrel Inc., East Norwalk, CT, USA. Geeignete Schmelzepumpen sind aber auch erhältlich von Robatech, Muri, Schweiz; Kreyenborg, Münster, Deutschland; Witte, Uetersen, Deutschland oder anderen Herstellern aus der KunststoffIndustrie.

Erfindungsgemäss vorgeschlagen ist ein Dosiersystem, welches hochviskose heisse Schmelzen zeitverzugslos und mit direkter Anbindung an einen Extruder oder einer Schmelzepumpe in Kapselhälften dosieren kann. Erfindungsgemäss für die meisten API geeignet sind Temperaturen unter 150°C, bevorzugt unter 120°C, bevorzugt unter 90°C und eine Verarbeitungszeit vom Dosieren bis zur Einbringung in die Kapsel von weniger als 30 Minuten, bevorzugter weniger als 10 Minuten, noch bevorzugter weniger als 3 Minuten.

Der Fachmann wird für diese Bedingungen die Stoffkomposition einer Schmelze mit der niedrigsten Viskosität auswählen. Die erfindungsgemässe Anordnung garantiert aber einen gegenüber der herkömmlichen Technologie der Weichkapselherstellung wesentlich höheren Spielraum für die Viskosität, aber auch für die verwendbaren Stoffkomponenten (Monomere und Polymere).

Erfindungsgemäss bevorzugt wird die Kapsel nach der Herstellung schockgekühlt, um Füllgut glasig zu machen. Dies erfolgt vorzugsweise durch kalte Gase (Stickstoff, Luft, CO₂) oder durch ein kaltes Bad aus mit der Hülle verträglichen Flüssigkeiten.

Mit der vorliegenden Erfindung gelingt es, die aus der Herstellung von schmelzextrudierten Dosierformen (z.B. Metrex) bekannten galenischen Vorteile (insbesondere Retardierung und molekulare Dispergierbarkeit) auf Kern/Schale-Strukturen und damit vollständig und gleichmässig überzogene Darreichungsformen, also Weichkapseln zu übertragen. So können die Vorteile der Schmelzextrusionstechnik für die Dosierung bestimmter API mit den Vorteilen vollständig umhüllter Darreichungsformen (Verkapselung) kombiniert werden. Insbesondere ist durch die Verwendung thermoplastisch geformter Filme für Kapselhüllen für Füllgüter hergestellt mit Schmelzextrusionstechnologie z.B. auch die Verwendung von Acrylaten möglich. Dies erlaubt beispielsweise die Formung von Kapseln mit einer Hülle aus magensaftresistenten Acrylaten und Retardierung des Füllgutes durch Acrylate oder andere Polymere in einem Schritt.

Es ist aber auch die Verwendung von besonders wasserarmer, pH abhängig sich auflösender oder durch Verdauungsenzyme oder generell enzymatisch an definierter Stelle spaltbarer Thermoplasten für Hülle und Füllgut denkbar.

Mit der vorliegenden Erfindung können Weichkapseln, umfassend eine Hülle und ein Füllgut mit mindestens einem pharmakologisch oder physiologisch aktiven Stoff, prinzipiell so hergestellt werden, dass die Hülle aus durch Schmelzextrusion thermoplastisch verarbeitbarem polymerem Material mit bis zu 250°C Siegeltemperatur aufgebaut ist und das geschmolzene Füllgut bei erhöhter Temperatur zwischen 50-180°C, aber unterhalb der Siegeltemperatur des Hüllenmateriales, mit Viskositäten von bis zu 100'000 mPa·s befüllt wird. Die Grenzen der Viskosität bei Verarbeitungstemperatur sind nach unten ausschliesslich durch die Toleranz der Schmelzextrusionsgeräte (bei Extrudern z.B. dem Spalt zwischen Gehäuse und Schnecke) und nach oben durch die Zeit/Druck-Verhältnisse, die für die Kapsel-Befüllung zur Verfügung stehen, limitiert.

Mit der vorliegenden Erfindung wird eine Reihe von technologischen Hindernissen überwunden, die im bisherigen Stand der Technik noch nicht gelöst werden konnten:
- Schutz der Wirkstoffe vor schädlicher Wärme bei Ansatz/Herstellung;
- Verarbeitung hochviskoser Schmelzen;
- Kontinuierliche Formung und einstufiges Verfahren (im Gegensatz zu Spritzguss oder Kalandrierung oder Formung gefolgt von Coating);
- Anpassung der Verarbeitungstemperaturen von Hülle und Füllmasse so, dass durch die nun mögliche Inkaufnahme höherer Viskositäten das Füllgut mindestens 20°C tiefer als die Hüllenmasse verarbeitet werden kann, so dass eine optimale Verschmelzung der beiden Hüllenbänder und damit eine durchgehend gleich dicke Umhüllung entsteht;
- Variable Einstellung des Freisetzungsverhaltens (Magensaftresistenz der Hülle, sofortige oder retardierte Freisetzung des Wirkstoffes);
- Vermeidung der Rekristallisation von API oder Füllgut-Matrix aus glasigen oder teilkristallinen Systemen;
- Weitgehende Vermeidung von Lösemitteln (niedermolekulare Stoffe mit hohem Dampfdruck), es sei denn, die Formulierung des Füllgutes erfordert die Anwesenheit von Lösemittel auf Grund der Löslichkeiten des API.

Gegenüber dem Stand der Technik ergeben sich gemäss der vorliegenden Erfindung folgende Vorteile:
- Die erfindungsgemässe Weichkapsel ist mechanisch flexibel und stabil. Die Kapseln sind allseitig umschlossen, können nicht auslaufen, und sind an der Hüllenaussenseite API-frei;
- Die erfindungsgemässen Weichkapselfüllgüter können leicht so formuliert werden, dass sie retardiert freigesetzt werden;
- Die mechanischen/chemischen Eigenschaften der Hülle der erfindungsgemässen Weichkapsel können leicht an verschiedene Eigenschaften des Füllgutes oder des Lagerklimas angepasst werden;
- Die Kapselhülle kann aus diversen thermoplastischen Materialien geformt werden, insbesondere auch aus magensaftresistenten Polymeren. Ein zusätzliches Coating entfällt bei den erfindungsgemässen Weichkapseln;
- Die Kapselhülle enthält vor und während der thermoplastischen Verarbeitung und Formung und während der Lagerung der Kapsel nur maximal soviel Wasser, wie das Polymermaterial auf Grund der Sorption bei der Lagerraumfeuchte aufnehmen kann. Die Füllgutzusammensetzungen verändern sich deshalb kaum durch Wasser- oder Weichmacherübertritt. Natürlich kann auch ein Polymer eingesetzt werden, welches getrocknet wurde, oder dem die für die Plastizität oder mechanischen Daten bei Gebrauch notwendigen Mengen an Weichmacher oder Lösemittel oder Wasser zugesetzt wurden;
- Es ist möglich, nicht nur Füllgutmatrizes auf der Basis von Polymeren, sondern auch auf Basis von schnell wasserlöslichen, kleinen Molekülen, die schmelzen und glasig, kristallin oder teilkristallin erstarren, zu verwenden;
- Es ist möglich, die Interaktion von Hülle und Füllgut zu eliminieren, da mit zunehmender Festigkeit und zunehmender Einheitlichkeit der Phasen des Füllgutes insbesondere auch der Austausch von Weichmacher oder andern kleinen Molekülen (Diffusion) abnimmt;
- Die Molekulardispersität von Füllgütern kann mit zunehmender Festigkeit und zunehmender Einheitlichkeit der Phasen erhalten werden. Die Rekristallisation des Wirkstoffes wird verhindert.

Mit der vorliegenden Erfindung sind neuartige Kombinationen von Hüllmaterial und Füllgut möglich, die bisher aus verfahrenstechnischen Gründen nicht realisiert werden konnten. Da mit der erfindungsgemässen Vorrichtung auch hochviskose Füllgüter bei höheren Temperaturen verkapselt werden können, ist auch der Einsatz anderer Hüllmaterialien möglich.

Das Hüllmaterial ist aus durch Schmelzextrusion thermoplastisch verarbeitbarem polymerem Material aufgebaut. Unter Schmelzextrusion im Sinne der vorliegenden Erfindung ist eine Herstellung, Verformung und Verschweissung einer thermoplastischen Masse bei Drücken von mehr als 1 bar (vorzugsweise mehr als 10 bar, noch bevorzugter mehr als 100 bar) und Temperaturen von mehr als 60°C, bevorzugter mehr als 80°C, noch bevorzugter mehr als 100 °C zu verstehen.

Bei der Schmelzextrusion wird ein Band aus dem entsprechenden Material geformt, indem das Material durch eine Breitschlitzdüse oder mit Hilfe von Blasverfahren in Filmform bei Viskositäten gebracht wird, die eine Verarbeitung unter Ausnutzung der Schwerkraft (Casting) nicht erlauben. Bei der Schmelzextrusion müssen daher notwendigerweise die Temperatur (um mindestens 20-50°C) sowie der Druck (auf mehr als 10 oder sogar mehr als 100 bar) gegenüber Umgebungsbedingungen erhöht werden. Gegebenenfalls kann dem thermoplastisch verarbeitbaren Material Weichmacher und/oder Wasser sowie gängige Additive wie beispielsweise Gleitmittel zugegeben werden. Die Schmelzextrusion kann in einem Doppelschnecken-Extruder oder Einschnecken-Extruder durchgeführt werden, wie sie dem Fachmann hinlänglich bekannt sind.

Erfindungsgemäss kann das durch Schmelzextrusion thermoplastisch verarbeitbare polymere Material beispielsweise ausgewählt werden aus der Gruppe bestehend aus Polyvinylpyrrolidon, Celluloseester und celluloseether, Polyvinylalkohol, Polyvinylalkoholacetalen, Polethylenglycol-Polyvinylalkohol-Pfropfpolymer, Polyacrylsäure (Carbopol),Polyacrylsäure-ester, Polyoxyethylen (Polyox) Polyosypropylene, Stärke, Polymilchsäure, Polylactic acidcoglycolide, Gelatine, Carrageenan, Casein, Gluten und deren Mischungen.

Beispiele für erfindungsgemäss geeignete Materialien sind die in der EP 1 103 254 A1 offenbarten stärkehaltigen Massen sowie die in der EP 1 586 436 A1 offenbarten Hüllmaterialien, wobei im letzteren Fall Hüllmaterialien auf der Basis von PVACL bevorzugt sind.

Erfindungsgemäss können auch die in der EP-1 586 436 A1 beschriebenen thermoplastischen Polymere eingesetzt werden, die ausgewählt sind aus der Gruppe bestehend aus Polyvinylalkohol, Celluloseether, Polycaprolacton, Polyamiden, Polyacrylsäure, Polyvinylpyrrolidon, Polymilchsäure oder Polyvinylalkoholacetalen (PVACL), Derivaten oder Gemischen derselben. Auf die entsprechende Offenbarung der EP-1 586 436 A1 wird ausdrücklich Bezug genommen.

Weiter sind alle thermoplastisch verarbeitbaren Polymere mit einer ausreichenden Fliess- und Siegeltemperatur zwischen 80 und ca. 250°C, sowie ausreichenden Festigkeit und Zugdehnung nahe der Siegeltemperatur und ausreichender Flexibität und Schlagzähigkeit als Hüllenmaterial einsetzbar, unabhängig davon ob sie wasserlöslich sind oder nicht.

Als für die orale Anwendung am Menschen interessante Polymere können (ohne abschliessende Aufzählung) zusätzlich zu oben aufgezählten Gruppen oder Einzelvertreter gelten Hydroxypropylcellulose (HPC), Hydroxypropylmethylcellulose (HPMC), Ethylcellulose (EC), Celluloseacetatphtalat (CAP), Hydroxypropylmethylcellulosephtalat (HPMCP), Hydroxypropylmethylcelluloseacetatsuccinat (HPMCAS, Aqoat), Natriumcarboxymethylcellulose (CMC), Polyvinylpyrrolidon (PVP), Polyvinylpyrrolidonvinylacetat-Copolymer (Kollidon VA), Polyvinylalkohol-polyethyleneglycol-Pfropfpolymer (Kollicoat IR), Carbopol (Polycarbophil), Methacrylsäure-Copolymer (Eudragit), Methacrylsäure-Ethylacrylat-Copolymer (Eudragit), Aminoalkylmethacrylat-Copolymere (Eudragit), Polyethylenglycol, Polyox, Maisprotein (Zein), Weizenprotein, und Sojaprotein (Gluten).

Erfindungsgemäss bevorzugt besteht die Hülle aus einem thermoplastischen, bei 37°C wasserlöslichen Polymer für die orale Anwendung im pharmazeutischen oder Nahrungsmittelbereich.

Erfindungsgemäss weiterhin bevorzugt besteht die Hülle aus magensaftresistenten oder sich pH-abhängig lösenden Polymeren.

Aus diesen Materialien können durch Schmelzextrusion Filme erzeugt werden, welche in üblichen Weichkapsel-Herstellungsverfahren wie beispielsweise dem Rotary-Die-Verfahren zu Weichkapseln verarbeitet werden können. Damit ergeben sich bedeutende Vorteile gegenüber dem bisherigen Stand der Technik der Weichkapseln, wo zur Erzielung bestimmter Hülleneigenschaften wie Magensaftresistenz die geformte Weichkapsel in einem zweiten Schritt mit einem funktionellen Polymer überzogen werden muss.

Im Gegensatz zum Hüllenmaterial, für das bei Verarbeitungstemperatur (Zuführung, Dehnung bei Befüllung, Versiegelung) bestimmte Mindestanforderungen an die mechanischen Eigenschaften gestellt werden, ist für die geschmolzene Füllgutmasse lediglich zu beachten, dass bei der Einbringung in die Weichkapselhälfte die Temperatur der Masse unterhalb der Verarbeitungstemperatur des Bandes liegt. Die übrigen mechanischen Daten, mit Ausnahme einer maximalen Viskosität, spielen keine wesentliche Rolle.

Die Formulierung des Füllmateriales wird also so gewählt, dass eine für die Dosierung maximale Viskosität bei der Verarbeitungstemperatur des Bandes nicht überstiegen wird. Dazu ist die Auswahl der geeigneten Stoffe (Polymere, Polymer/Monomer Gemische) bzw. die Auswahl der richtigen Kettenlängen eines Polymers ausreichend.

Für die Verwendung als orale Darreichungsform im Bereich der Ernährung oder Pharmazie sollen die erfindungsgemässen Füllgüter bei Kontakt mit Wasser eine ausreichende potentielle Bioverfügbarkeit zeigen. Unter "potentiell ausreichender Bioverfügbarkeit" wird ein der pharmakologischen Wirkung und den physiologischen Gegebenheiten angepasstes sofortiges oder verzögertes molekulardisperses Angebot des API im Magen-Darmtrakt verstanden. Bei guter Wasserlöslichkeit des Stoffes sollen mindestens 80% des API in der geforderten Zeit in den Flüssigkeiten des Magen-Darmtraktes gelöst sein, was als Simulation mit der sogenannten Freisetzung gemessen werden kann. Für schlecht wasserlösliche oder lipophile, mit Wasser nicht mischbare API wird darunter die Emulgierung von mindestens 80% des API in Form von Tröpfchen, kolloidalen oder anderen komplizierten Strukturen mit einer Oberfläche von mindestens dem 1000-fachen der ursprünglichen "Kapselfüllgutoberfläche" verstanden.

Der Übergang vom "festen" in den "plastischen" oder "flüssigen" Zustand kann bei niedermolekularen und kristallisierenden Substanzen mit dem sogenannten Schmelzpunkt (Smp.) beschrieben werden. Als Schmelztemperatur bezeichnet man die Temperatur, bei der ein Stoff schmilzt, das heisst vom festen in den flüssigen Aggregatzustand übergeht. Die Schmelztemperatur ist abhängig vom Stoff, im Gegensatz zur Siedetemperatur aber nur sehr wenig vom Druck (Schmelzdruck) abhängig. Schmelztemperatur und Schmelzdruck werden zusammen als Schmelzpunkt bezeichnet, wobei dieser den Zustand eines Reinstoffes beschreibt und Teil der Schmelzkurve im Phasendiagramm des Stoffes ist. Für reine Stoffe ist der Schmelzpunkt identisch mit dem Gefrierpunkt und bleibt während des gesamten Schmelzvorganges konstant.

Für Polymere, amorphe, oder glasige Stoffe ist die Übergangstemperatur oder Glastemperatur charakteristisch. Die Glasübergangs- oder Erweichungstemperatur (Tg) ist die Temperatur, bei der ein Glas die größte Änderung der Verformungsfähigkeit aufweist. Dieser so genannte Glasübergang trennt den unterhalb liegenden spröden energieelastischen Bereich (=Glasbereich) vom oberhalb liegenden weichen entropieelastischen Bereich (=gummielastischer Bereich). Der Übergang in den Fließbereich des amorphen Kunststoffs ist fließend. Teilkristalline Kunststoffe besitzen sowohl eine Glasübergangstemperatur, unterhalb derer die amorphe Phase 'einfriert' (einhergehend mit Versprödung), als auch eine Schmelztemperatur, bei der sich die kristalline Phase auflöst. Die Schmelztemperatur trennt den entropieelastischen Bereich deutlich vom Fließbereich ab. Kristalline Kunststoffe weisen im Gegensatz dazu nur eine Schmelztemperatur auf.

Für Gemenge und Gemische aller Arten eignet sich zur Beschreibung des Verhaltens der Viskosität in Abhängigkeit der Temperatur zum Beispiel der melt flow index. Die Schmelzindexmessung ist eine technologische Prüfmethode zur schnellen, qualitativen Bestimmung der Fließeigenschaften von thermoplastischen Formmassen. Der Schmelzindex stellt eine Mass für die Fließfähigkeit der Polymerschmelze bei der gewählten Temperatur dar und ist in erster Linie durch die mittlere Molmasse bestimmt. Der Schmelzindex (MFI = Melt Flow Index) in g/10 min bei Temperatur T wird dabei als diejenige Masse definiert, welche innerhalb eines Zeitraumes von 10 Minuten bei einer festgelegten Kolbenkraft und einer bestimmtem Massetemperatur T durch eine genormte Düse gedrückt wird.

Im Gegensatz zum Stand der Technik konnten bisher molekulardisperse Füllgutsysteme nur in Kapseln gebracht werden, wenn sie bei der Temperatur der Versiegelung des Hüllenmateriales noch pumpbar waren. Die Erhöhung der Siegeltemperatur der Hüllenmaterialien, bedingt durch die neu mögliche thermoplastische Verarbeitung der Hüllenpolymere zu Filmen, ermöglicht die Andienung von höherschmelzenden (>51°C) und höherviskosen Massen. Der Fachmann erhält für die Formulierung eines Füllgutsystems enthaltend mindestens ein API wesentlich mehr Spielraum in der Auswahl der Stoffe. Die Abhängigkeit von Temperatur und Viskosität erlaubt, die Entwicklung der Füllmasse nach dem einen ODER andern Parameter auszurichten.

Als nicht einschränkende von erfindungsgemäss hergestellten Weichkapseln seien genannt:

### Beispiel 1:

| | |
|---|---|
| Gelucire 50/13 (Lauroyl macrogolglycerides,mp 44°C) | 262.5 mg |
| Lutrol F127 (Pluronic F127, poloxamer 407, mp 57°C) | 262.5 mg |
| Diclofenac Natrium (mp 284°C) | 75.0 mg |

Die viskose Schmelze-Suspension wurde in Weichkapseln mit einer Hülle aus PVA/Stärke bei eine Extruder-Düsentemperatur von 75°C und einer Viskosität von 5000 mPa·s gefüllt; man erhielt Kapseln mit verzögerter Freisetzung (retardiert) mit einem weissen festen Inhalt. Die Partikelgrössenverteilung des Diclofenac blieb praktisch unverändert.

### Beispiel 2:

| | |
|---|---|
| Ascorbinsäure (m.p./dec. 190°C) | 180 mg |
| Klucel EF (Hydroxypropylcellulose)(Erw. T.>130°C) | 180 mg |
| Sorbitol (Sorbidex P16616 /Cargill)(m.p. 166-168°C) | 50 mg |
| Triethylcitrat | 40 mg |

Die Mischung liess sich bei 130-140°C zu einer teigartigen, weissen opaken Masse verarbeiten, die eine recht hohe Viskosität zeigte (ca. 100'000 mPa·s), und in 7.5 minims Weichkapseln mit einer Hülle aus PVA/Stärke bei einer Extruder-Düsentemperatur von 110°C gefüllt wurde. Man erhielt Kapseln mit verzögerter Freisetzung (retardiert).

### Beispiel 3:

| | |
|---|---|
| Ibuprofen (mp 74-77°C) | 210 mg |
| Kollidon K30 (Polyvinylpyrrolidon)(Erw. T. >150°C) | 260 mg |
| Isomalt (mp 145-150°C) | 47 mg |
| Wasser | 3 mg |

Im Extruder wurden die vorstehenden Komponenten zu einer klaren homogenen viskosen Schmelze von ca. 20'000 bis 50'000 mPa·s (ca. 110°C) bei maximal 120°C geschmolzen und mit einer Düsentemperatur von 90°C in 10 minims oval Stärkekapseln gefüllt. Es wurden ca. 80% Ibuprofen in 45 min freigesetzt (900 ml Wasser, 37°C, paddle 100 rpm).

Die vorliegende Erfindung wird nachstehend anhand von bevorzugten, nicht einschränkenden Ausführungsformen und Zeichnungen erläutert. Es zeigen:
- Figur 1: eine herkömmliche Rotary-Die-Vorrichtung,
- Figur 1a: ein Detail der Vorrichtung gemäss Figur 1,
- Figur 2: eine schematische Darstellung einer erfindungsgemässen Rotary-Die-Anlage zur Verarbeitung von viskosen Schmelze,
- Figur 3: eine perspektivische Darstellung eines Füllkeils mit eingebautem Portionierer,
- Figur 4: eine schematische Darstellung der Steuerung eines Portionierers,
- Figur 5: eine stark vereinfachte Darstellung eines Portionierers mit Ventilnadeln,
- Figur 6a und 6b: stark vereinfachte Darstellungen eines Portionierers mit Dosierrohr in Öffnung- und Schliessposition,
- Figur 7: eine schematische Darstellung einer Rotary-Die-Vorrichtung mit einem als Dosierungshohlwelle ausgebildeten Portionierer,
- Figur 8: die Vorrichtung gemäss Figur 7 mit weiteren Einzelheiten,
- Figur 9: einen Querschnitt durch einen Füllkeil mit Y-förmigem Verteilkanal und permanent rotierender Dosierungshohlwelle,
- Figur 10: einen Querschnitt durch einen Füllkeil mit direkt zu den Keilwänden führenden Verteilkanälen und oszillierender Dosierungshohlwelle,
- Figur 11: einen Querschnitt durch einen Füllkeil mit einem Verteilkanal zur Keilspitze, und
- Figur 12: einen Querschnitt durch einen Füllkeil mit unmittelbar an die Keiloberfläche angrenzende Abgabeöffnungen der Dosierhohlwelle.

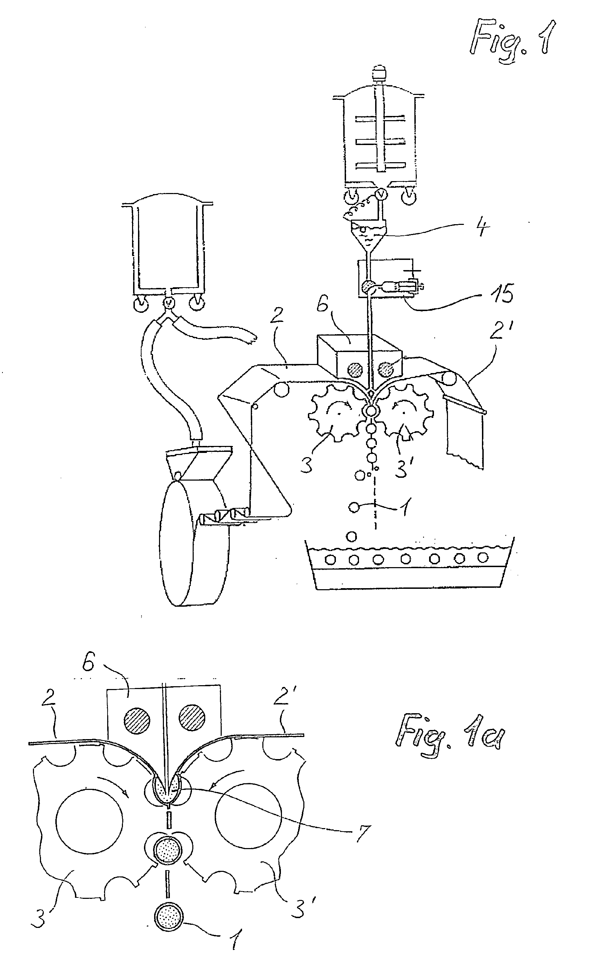

Figur 1 zeigt eine herkömmliche Rotary-Die-Vorrichtung mit einer konventionellen Dosierkolbenpumpe 15 als Fördermittel für das Füllgut 4. Das Füllgut gelangt über die Dosierkolbenpumpe zum Füllkeil 6, unter dem zwei endlose Filmbänder 2, 2' beispielsweise aus Gelatine-Lösung an den beiden Formwalzen 3, 3' zusammengeführt und zu Kapseln geformt werden. Vor dem Verschliessen und Abquetschen der Kapseln 1 wird das Füllgut in den Kapselhohlraum 7 eingeführt (Figur 1a). Die Dosierkolbenpumpe 15 ist im Abstand zum Füllkeil 6 angeordnet und sie dosiert taktweise ein Volumen entsprechend dem gewünschten Füllgrad der Kapsel. Das dosierte Füllgut muss dabei eine Strecke von ca. 50 cm überwinden, bevor es an den eigentlichen Ort der Kapselbefüllung gelangt. Ausserdem legt das Füllgut diese Strecke als taktweise vorgeschobene Säule zurück, was sich wie einleitend beschrieben bei hochviskosen Flüssigkeiten unter hohen Temperaturen negativ auf das Stabilitätsverhalten, die Dosiergeschwindigkeit und die Dosiergenauigkeit, sowie den benötigten Druck und mechanische Stabilität der Bau-Komponenten auswirkt.

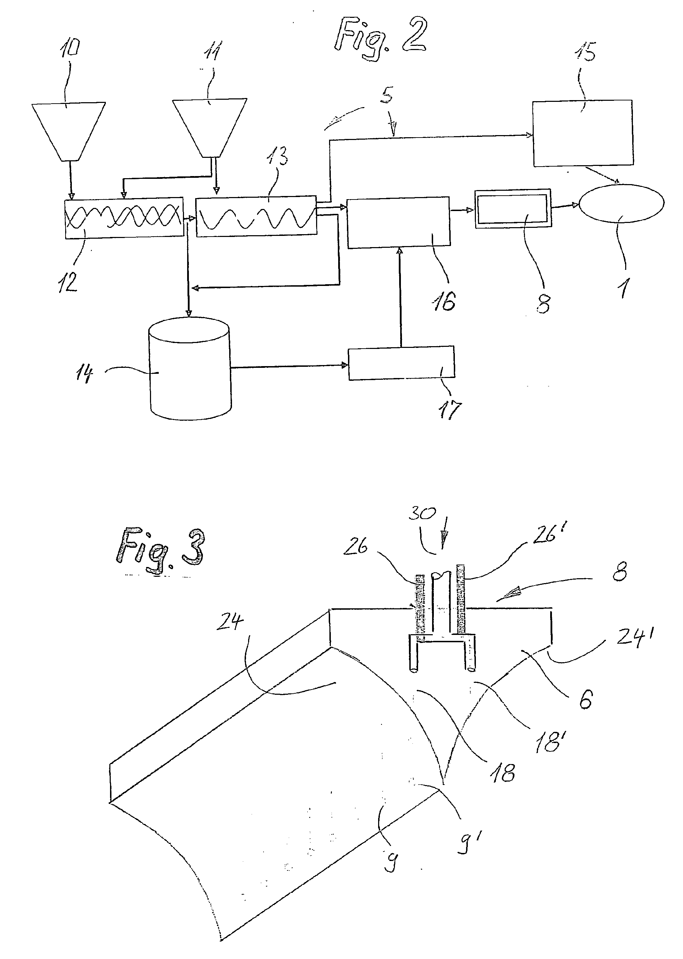

In Figur 2 ist schematisch eine Gesamtanlage für einen erfindungsgemässen Rotary-Die-Prozess dargestellt. In einer konventionellen Dosier- bzw. Mischvorrichtung 10 werden die Pulver- oder Flüssigkeitskomponenten der inaktiven Matrix aufbereitet und in einer Schmelz- und Mischanlage 12 (wie z.B. einem Zweiwellen-Extruder) homogen aufbereitet. Der API kann ebenfalls auf dieser Stufe über einen Mischer oder Dosierer zugegeben werden.

Das entstehende Produkt kann geformt, geschnitten, gekühlt und in einem Zwischenlager 14 zwischengelagert werden, von wo es in einer späteren Phase wieder durch eine konventionelle Misch-Schmelzvorrichtung 13 (z.B. einen Ein- oder Zweiwellen-Extruder) oder einer melt-on-demand Anlage 17 zur Schmelze aufbereitete werden kann. Logistisch besonders vorteilhaft ist das wieder Aufschmelzen solcher Füllgutmassen mit Hilfe eines Melt-On-Demand-Systems 17, wie es kommerziell z.B. von Robatech, Muri, Schweiz oder ITW Dynatec, 31 Volonteer Drive, Hendersonville TN, USA erhältlich ist.

Alternativ kann die feste Matrix ohne API aus dem Zwischenlager 14 in der Schmelz-Mischanlage 13 aufgeschmolzen und API z.B. in kristalliner oder generell partikulärer Form aus dem Mischer/Dosierer 11 zugeknetet werden und zu einer homogenen Schmelze oder aber auch zu einer Suspensions-Schmelze verarbeitet werden. Der Fachmann kann die Bauweise der Extruder und deren Betriebsbedingungen entsprechend deen gewünschten Anwendungsbedingungen auswählen. So kann auch ein Erhalt der Kristallinität der API sichergestellt werden. Besonders bevorzugt wird der aktive Stoff in kontrollierter Korngrösse oder mikropartikulärer Form (mikronisiert, pelletisiert oder gecoated) der Schmelze zugesetzt.

Das am Ende von 13 oder 17 entstandene, gewünschte, viskose Material wird direkt über eine konventionelle Dosierpumpe 15 in die Kapsel 1 oder alternativ über eine Schmelzepumpe 16 über den Dosierer 8 in die Kapsel 1 dosiert. In einer besonders bevorzugten Ausführungsform wird einem oder mehreren Extrudern oder Melt-On-Demand-Systemen eine Schmelzpumpe 16 mit Differenzialdruck-Kontroller nachgeschaltet. Diese stellt sicher, dass keine Druckschwankungen auftreten, womit ein ausgesprochen gleichförmiger Strom erzielt werden kann. Solche Schmelzpumpen sind kommerziell erhältlich z.B. Harell Inc., East Norwalk, CT, USA. Geeignete Schmelzpumpen sind aber auch erhältlich bei Robatech, Muri, Schweiz oder bei anderen Herstellern von Maschinen der Kunststoffindustrie.

Der Fachmann wird die für die gestellte Aufgabe der exakten Dosierung einen optimalen Prozess aus den aufgezeigten Varianten auswählen. Dazu stehen alle die aus der Kunststoff- oder Heiss-Klebstoff-Industrie bekannten Geräte im Prinzip zur Verfügung.

Bei der Anlage gemäss Figur 2 können ersichtlicherweise sowohl die Extruder 12 und 13, als auch die Schmelzpumpe 16 die Funktion eines allgemein mit 5 bezeichneten Fördermittels erfüllen, um einen möglichst gleichförmigen und druckschwankungsfreien Materialstrom zum Portionierer 8 zu fördern. Fördereinrichtungen, die nach dem Hubverdrängungsprinzip arbeiten wie z.B. Kolbenpumpen, sind dazu nicht geeignet. Eine konventionelle Dosierkolbenpumpe 15 kann aber trotzdem in das System integriert sein, um für schnelle Schmelz- und Mischvorgänge und niedrigviskose Massen das Füllgut auf konventionelle Weise und ohne Portionierer 8 zu Kapseln 1 zu verarbeiten.

Figur 3 zeigt schematisch einen Füllkeil 6, bei dem der Portionierer 8 als Doppelschieber mit den beiden vertikal bewegbaren Schieberelementen 26, 26' ausgebildet ist. Dabei wird eine zentrale Zuleitung 30 in separate Verteilkanäle 18, 18' aufgeteilt, wobei diese Verteilkanäle durch die Schieberelemente 26, 26' wechselweise geöffnet und geschlossen werden können. Die Verteilkanäle 18, 18' führen zu Füllgutaustrittöffnungen 9, 9' an den beiden gegenüberliegenden Keilflächen 24, 24'. An der zentralen Zuleitung 30 wird die Schmelze mit konstantem Druck zugeführt.

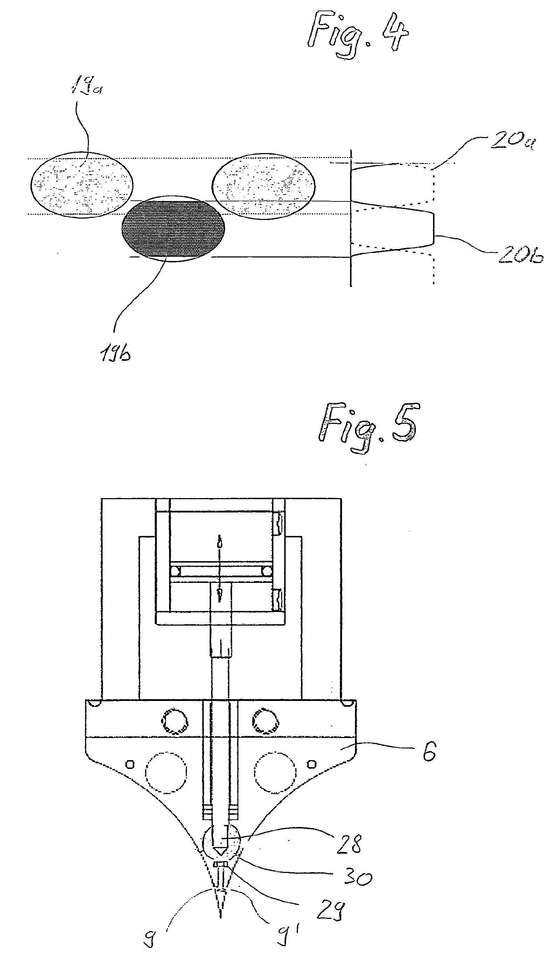

Die Öffnungszeiten und die Öffnungsquerschnitte an den Schieberelementen werden so gewählt, dass der Volumenstrom bei gleich bleibendem Druck proportional zur dosierten Menge ist, die durch die Austrittsöffnungen 9 in die Kapsel fliesst. Um keine Druckschwankungen zu erzeugen und um den viskosen Volumenstrom langsam zu durchschneiden, verlaufen die jeweils frei gegebenen Öffnungsquerschnitte nacheinander abgefüllter Kapseln etwa sinusartig und zwar sich sinusartig überlagernd. Diese Situation ist in Figur 4 schematisch dargestellt. Die Öffnungsquerschnitte 19a, 19b überlagern sich, wobei zwei gegenläufige und sich ebenfalls überlagernde Sinuskurven 20a, 20b entstehen. Die Amplitude dieser Kurve entspricht jeweils dem maximal freigegebenen Querschnitt. Ersichtlicherweise wird bei einer derartigen Steuerung der Öffnungsquerschnitt der einen Reihe kontinuierlich verkleinert, während der Öffnungsquerschnitt der folgenden Reihe bereits geöffnet wird. Eine derartige Steuerung der Schieberelemente kann beispielsweise über eine Nockenwelle erfolgen.

Figur 5 zeigt ein Ausführungsbeispiel eines Füllkeils 6, bei dem der Portionierer mehrere, der Anzahl zu befüllenden Kapseln entsprechende und getaktete Ventilnadeln 28 aufweist, die mit entsprechenden Ventilsitzen 29 zusammenwirken. Die Ventilnadeln sind über dem Füllkeil gelagert und sie bewegen sich in der Symmetrieachse des Füllkeils in senkrechter Richtung. Die zentrale Zuleitung 30 fördert die Schmelze unter gleichmässigem Druck im rechten Winkel zu den Ventilnadeln. Vom Ventilsitz 29 führt ein zentraler Verteilkanal zu den Austrittöffnungen 9, 9' (Y-Kanal). Auch die Ventilnadeln werden bevorzugt entsprechend einer Sinuskurve geöffnet und geschlossen und beispielsweise über eine Nockenwelle oder über elektronisch gesteuerte Pneumatik oder Hydraulik betätigt.

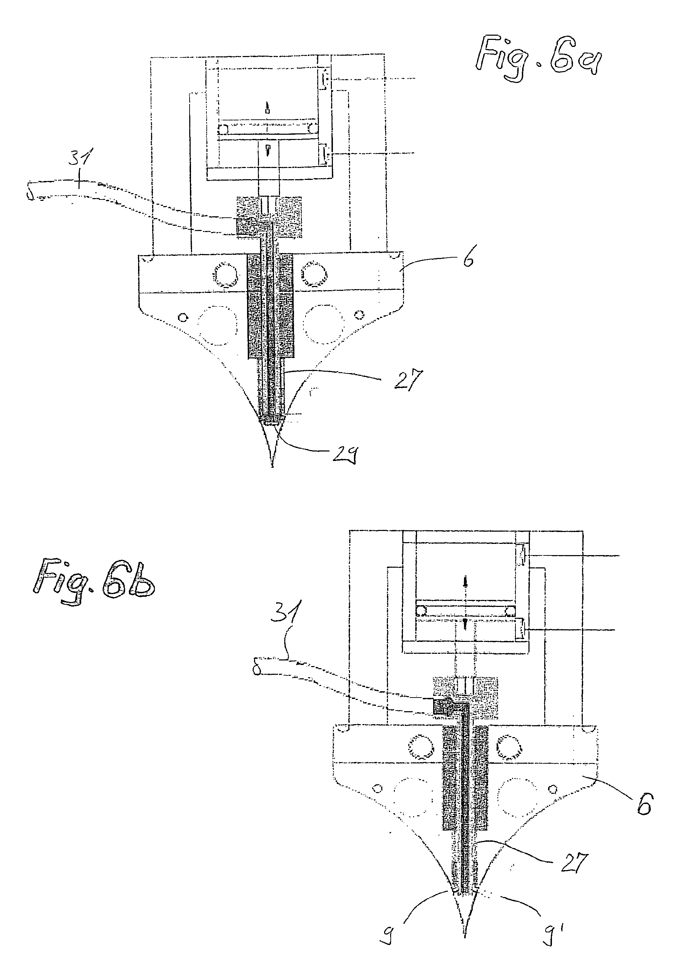

In den Figuren 6a, 6b ist ein weiteres Ausführungsbeispiel eines Portionierers dargestellt, der nach dem Schieberprinzip arbeitet. Anstelle der Ventilnadeln sind auf die gleiche oder ähnliche Weise ansteuerbar mehrere Dosierrohre 27 in einer Reihe im Füllkeil gelagert. Die vom Fördermittel kommende Schmelze wird beispielsweise über flexible Leitungen 31 in die Dosierrohre eingeleitet. Das untere freie Ende jedes Dosierrohrs wird taktweise gegen einen Ventilsitz 29 gepresst und damit verschlossen, wie in Figur 6a dargestellt. Sobald das Rohr angehoben wird, fliesst Füllgut über sehr kurze Kanäle zu den Füllgutöffnungen 9, 9'. Die Öffnungsposition ist in Figur 6b dargestellt.

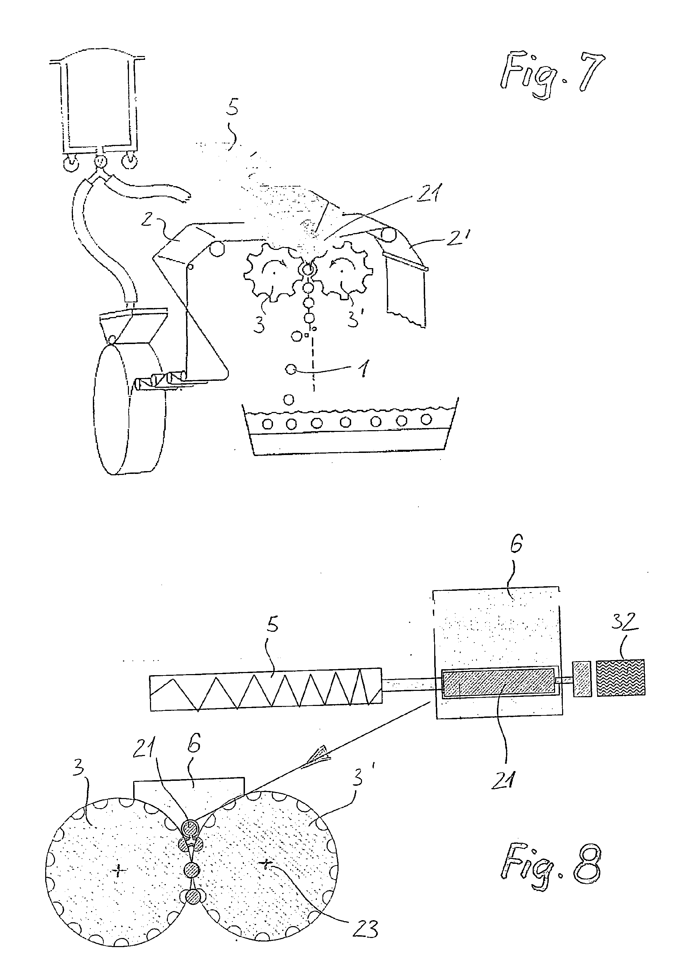

Beim Ausführungsbeispiel gemäss den Figuren 7 und 8 besteht der Portionierer aus einer Dosierungshohlwelle 21, welche parallel zu den Drehachsen 23 der Formwalzen 3, 3' drehbar im Füllkeil gelagert ist. Wie nachstehend noch genauer dargestellt, ist diese Dosierungshohlwelle an ihrem Mantel mit Ausgabeöffnungen versehen und sie steht in unmittelbarer Verbindung mit dem Fördermittel 16, 13 oder 17 für die druckschwankungsfreie Zufuhr der Schmelze. Die Dosierungshohlwelle 21 kann beispielsweise über einen Elektromotor 32 kontinuierlich im gleichen Drehsinn oder oszillierend in beide Drehrichtungen bewegt werden. Der Antriebsmotor ist dabei auf der von der Zufuhrleitung für die Schmelze abgewandten Seite angeordnet. Für den Anschluss an das Fördermittel 5 sind entsprechende Gleitringdichtungen erforderlich, um die Drehbewegung zu ermöglichen.

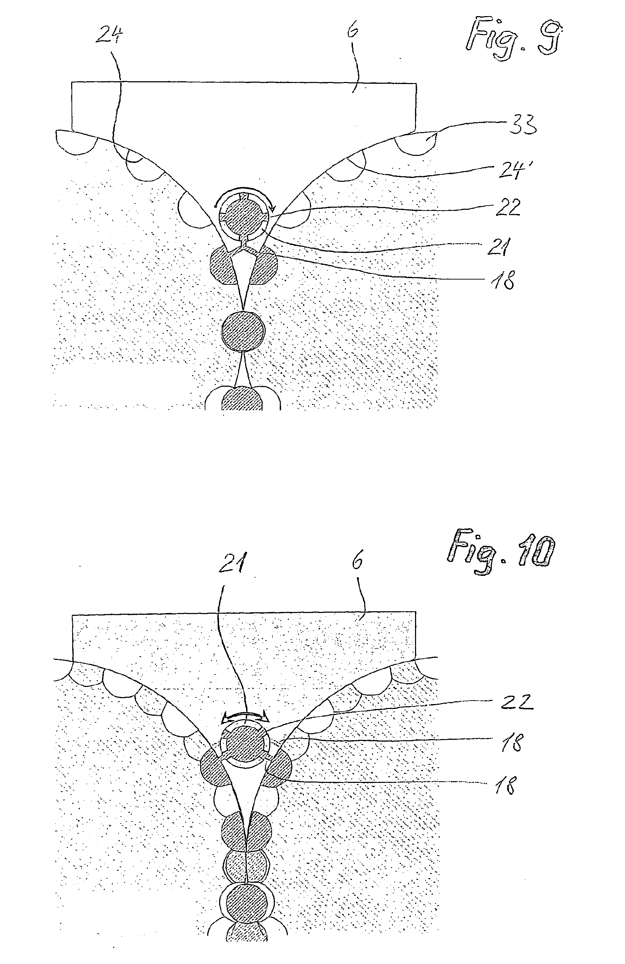

Ein erstes Ausführungsbeispiel einer Dosierhohlwelle 21 ist in Figur 9 dargestellt. Die das Füllgut führende Dosierhohlwelle ist mit vier in regelmässiger Winkelteilung angeordneten Ausgabeöffnungen 22 versehen. Bei einer gleichsinnigen Drehung der Dosierhohlwelle 21 korrespondieren diese Öffnungen in einer Winkelstellung mit je einem Verteilkanal 18, der Y-förmig zu separaten Austrittöffnungen führt, welche auf der gleichen Höhe liegen und welche je eine Kapselhälfte befüllen. Durch die Wahl des Öffnungswinkels bzw. Schlitzes, der richtigen Drehzahl und damit der Verweil- oder Überstreichzeit der Ausgabeöffnungen 22 über den Verteilkanälen 18, wird die Masse abhängig von der Viskosität und dem Druck portioniert. Der Durchmesser der Verteilkanäle 18 beträgt dabei mindestens 1 mm, vorzugsweise jedoch mindestens 2 mm und noch bevorzugter ein Drittel des Kapseldurchmessers bzw. ein Drittel der am Füllloch vorbeilaufenden Breite eines Napfs 33 in der Formwalze. Aus Gründen der besseren Übersichtlichkeit ist beim beschriebenen und bei den nachstehenden Ausführungsbeispielen das Filmband zwischen den Formwalzen und dem Füllkeil nicht dargestellt.

Ein weiteres Ausführungsbeispiel einer Dosierhohlwelle 21 ist in Figur 10 dargestellt. Dabei sind auf jeder Seite des Füllkeils zwei separate Reihen von Verteilkanälen 18 angeordnet, von denen jeder zu einem separaten, seitlich versetzten Napf an der Formwalze führt. Die Ausgabeöffnungen 22 sind nicht in gleichmässiger Winkelteilung, sondern paarweise zusammen angeordnet und die Dosierhohlwelle 21 bewegt sich nicht gleichsinnig, sondern oszillierend in beide Drehrichtungen. Dabei werden im Wechsel die einzelnen Verteilkanäle freigegeben.

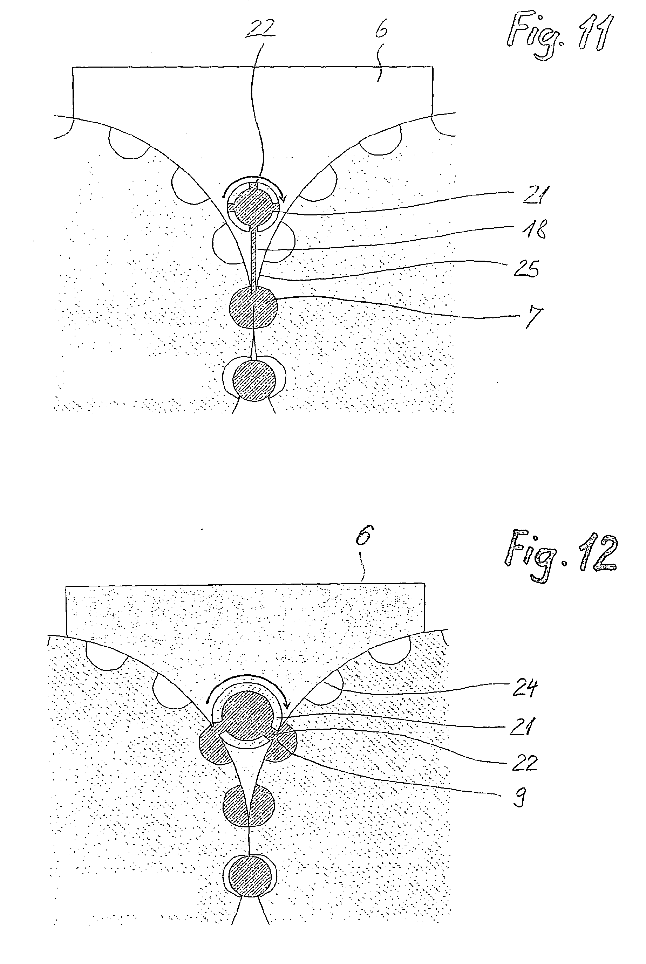

Beim Ausführungsbeispiel gemäss Figur 11 ist die Dosierungshohlwelle 21 gleich angetrieben und aufgebaut wie beim Ausführungsbeispiel gemäss Figur 9. Ein einzelner Verteilkanal 18 führt jedoch direkt zur Keilspitze 25 in den sich aufbauenden Kapselhohlraum 7. Dabei handelt es sich um eine so genannte Kopflochbefüllung. Die Dosierhohlwelle könnte anstelle einer kontinuierlichen Bewegung selbstverständlich ebenfalls in beide Drehrichtungen oszilliert werden.

Schliesslich zeigt Figur 12 noch ein Ausführungsbeispiel, bei dem faktisch die Verteilkanäle ganz wegfallen. Die Ausgabeöffnungen 22 an der Dosierungshohlwelle 21 fallen praktisch mit den Füllgutaustrittsöffnungen 9 in den Keilflächen 24 des Füllkeils zusammen. Bei dieser Lösung ist es nicht möglich, dass die Dosierungshohlwelle 21 durchgehend den gleichen Aussendurchmesser aufweist, weil sonst die Keilspitze nicht mit ausreichender mechanischer Stabilität mit dem restlichen Füllkeil verbunden werden kann. Hingegen kann durch Verjüngung der Dosierwelle zwischen den Dosierstellen eine Verschraubung der Füllkeilspitze mit dem Füllkeil-Körper erreicht werden.

Erfindungsgemäss bevorzugt wird die Kapsel nach der Herstellung schockgekühlt, um Füllgut glasig zu machen. Dies erfolgt vorzugsweise durch kalte Gase (Stickstoff, Luft, CO₂) oder durch ein durch kaltes Bad aus mit der Hülle verträglichen Flüssigkeiten.

Die erfindungsgemässen Weichkapseln können für alle herkömmlichen Zwecke eingesetzt werden, für welche Weichkapseln zur Anwendung gelangen. Beispielhaft seien die orale, rektale oder vaginale Applikation genannt, wobei die Kapsel als Nahrungsergänzungsmittel, Pharmazeutisches Produkt, Medizinal-Produkt oder für kosmetische oder technische Zwecke appliziert werden kann.

## Patentansprüche

1. Verfahren zur Herstellung von Weichkapseln (1) nach dem Rotary-Die-Prinzip, bei dem zwei kontinuierlich zusammengeführte Filmbänder (2, 2') zwischen zwei gegenläufig rotierende Formwalzen (3, 3') zu Kapseln geformt werden, wobei ein flüssiges Füllgut (4) mit Hilfe eines Fördermittels (5) unter Druck über einen Füllkeil (6) in den sich bildenden Kapselhohlraum (7) eingeführt wird, **dadurch gekennzeichnet, dass** das Fördermittel (5) wenigstens einen kontinuierlichen, vorzugsweise druckschwankungsfreien Füllgutstrom erzeugt, welcher durch einen getrennt vom Fördermittel, vorzugsweise innerhalb des Füllkeils angeordneten Portionierer (8) in Einzelportionen aufgeteilt wird, bevor diese den Ort (9) der Kapselfüllung erreichen.

2. Verfahren nach Anspruch 1, **dadurch gekennzeichnet, dass** das Füllgut im Fördermittel (5) zu einer viskosen Schmelze mit einer Temperatur von 51° bis 200°C aufbereitet wird.

3. Verfahren nach Anspruch 1 oder 2, **dadurch gekennzeichnet, dass** der Förderdruck und die Förderstrecke zwischen dem Fördermittel (5) und dem Ort (9) der Kapselfüllung derart dimensioniert werden, dass das Füllgut nach dem Verlassen des Fördermittels den Ort der Kapselfüllung innerhalb von weniger als 30 Minuten, vorzugsweise innerhalb von weniger als 10 Minuten und noch bevorzugter innerhalb von weniger als 3 Minuten erreicht.

4. Verfahren nach einem der Ansprüche 1 bis 3, **dadurch gekennzeichnet, dass** der Füllgutstrom durch den Portionierer (8) in wenigstens zwei Teilströme aufgeteilt wird, welche zu separaten Abfüllorten (9) führen, wobei die Aufteilung derart phasenverschoben erfolgt, dass die endgültige Kapselfüllung an den Teilströmen nacheinander erreicht wird und jeweils der zweite Teilstrom freigegebenen wird, bevor der erste Teilstrom unterbrochen wird.

5. Verfahren nach einem der Ansprüche 1 bis 4, **dadurch gekennzeichnet, dass** die Einzelportionen des Füllgutstroms zwischen dem Portionierer (8) und dem Ort (9) der Kapselfüllung über eine Distanz von 0 bis maximal 50mm, vorzugsweise bis maximal 30mm geführt werden.

6. Verfahren nach einem der Ansprüche 1 bis 5, **dadurch gekennzeichnet, dass** das Füllgut zuerst als Mischung von Matrixmaterial und aktiver Substanz aufgeschmolzen und danach im festen, zerkleinerten Zustand zwischengelagert wird, bevor es erneut aufgeschmolzen und über das Fördermittel dem Portionierer (8) zugeführt wird.

7. Verfahren nach einem der Ansprüche 1 bis 6, **dadurch gekennzeichnet, dass** die Förderung des Füllgutstroms zum Portionierer (8) mit einem Umlaufverdrängungsfördermittel, insbesondere mit einer Schraubenpumpe und bevorzugt mit einer Schmelzextrusionsvorrichtung erfolgt.

8. Verfahren nach einem der Ansprüche 1 bis 7, **dadurch gekennzeichnet, dass** der Füllgutstrom bei der Dosierung bei einer Temperatur von 0° bis 60°C, vorzugsweise bei 0° bis 50°C eine dynamische Viskosität von mehr als 5000 mPa·s oder bei einer Temperatur von mehr als 50°C eine Viskosität von wenigstens 10 mPa·s, bevorzugter von mehr als 1000 mPa·s, noch bevorzugter von mehr als 5000 mPa·s aufweist.

9. Vorrichtung zur Herstellung von Weichkapseln (1) nach dem Rotary-Die-Prinzip mit zwei gegenläufig antreibbaren Formwalzen (3, 3'), zwischen denen zwei kontinuierlich zuführbare Filmbänder (2, 2') zu Kapseln formbar sind, mit einem im Einzugsbereich der Formwalzen angeordnetem Füllkeil, zum Zuführen eines flüssigen Füllgutes (4) in den sich bildenden Kapselhohlraum (7), sowie mit einem Fördermittel (5) für die Druckbeaufschlagung des Füllguts, **dadurch gekennzeichnet, dass** mit dem Fördermittel wenigstens ein kontinuierlicher, vorzugsweise druckschwankungsfreier Füllgutstrom erzeugbar ist und dass der Füllgutstrom mit einem getrennt vom Fördermittel, vorzugsweise innerhalb des Füllkeils (6) angeordneten Portionierer (8) in Einzelportionen aufteilbar ist, welche dem Ort (9) der Kapselfüllung zuführbar sind.

10. Vorrichtung nach Anspruch 9, **dadurch gekennzeichnet, dass** das Fördermittel ein Umlaufverdrängungsfördermittel, insbesondere eine Schraubenpumpe und bevorzugt eine Schmelzextrusionsvorrichtung ist.

11. Vorrichtung nach Anspruch 9 oder 10, **dadurch gekennzeichnet, dass** zwischen dem Portionierer (8) und dem Ort (9) der Kapselfüllung an den Oberflächen des Füllkeils (6) Verteilkanäle (18) mit einer maximalen Länge von 50mm, vorzugsweise von 30mm angeordnet sind.

12. Vorrichtung nach einem der Ansprüche 9 bis 11, **dadurch gekennzeichnet, dass** der Füllgutstrom am Portionierer (8) in wenigstens zwei Teilströme aufteilbar ist, welche direkt oder über getrennte Verteilkanäle zu separaten Abfüllorten (9) leitbar sind, wobei die Teilströme phasenverschoben nacheinander erzielbar sind.

13. Vorrichtung nach Anspruch 12, **dadurch gekennzeichnet, dass** zum Speisen der beiden Teilströme am Portionierer (8) Öffnungsquerschnitte (18) für jeden Teilstrom entsprechend sich sinusartig überlagernder Kurven nacheinander freilegbar und wieder verschliessbar sind.

14. Vorrichtung nach einem der Ansprüche 9 bis 13, **dadurch gekennzeichnet, dass** der Portionierer (8) wenigstens eine Dosierhohlwelle (21) aufweist, welche drehbar im Füllkeil (6) gelagert ist und welche Ausgabeöffnungen (22) aufweist, welche in wenigstens einer Drehwinkellage mit einer Öffnung bzw. mit einem Kanal im Füllkeil (6) korrespondiert.

15. Vorrichtung nach Anspruch 14, **dadurch gekennzeichnet, dass** die Dosierungshohlwelle (21) parallel zu den Drehachsen (23) der Formwalzen gelagert ist und dass jede Ausgabeöffnung (22) entweder mit einem zu den Keilflächen (24, 24') oder mit einem zur Keilspitze (25) des Füllkeils (6) führenden Kanal verbindbar ist.

16. Vorrichtung nach einem der Ansprüche 9 bis 13, **dadurch gekennzeichnet, dass** der Portionierer (8) wenigstens einen Dosierschieber (26, 26') aufweist, der linear verschiebbar im Füllkeil (6) gelagert ist und mit dem taktweise Zufuhrkanäle für das Füllgut freilegbar bzw. verschliessbar sind.

17. Vorrichtung nach Anspruch 16, **dadurch gekennzeichnet, dass** der Dosierschieber senkrecht zu einer durch die beiden Drehachsen der Formwalzen verlaufenden Ebene gelagert ist.

18. Vorrichtung nach Anspruch 16 oder Anspruch 17, **dadurch gekennzeichnet, dass** der Dosierschieber als Dosierrohr ausgebildet ist, über den das Füllgut zuführbar ist.

19. Vorrichtung nach einem der Ansprüche 9 bis 13, **dadurch gekennzeichnet, dass** der Portionierer (8) wenigstens eine Ventilnadel (28) aufweist, die mit einem Ventilsitz (29) im Füllkeil zusammenwirkt.

## Claims

1. A method for producing soft capsules (1) on the rotary die principle, in which two continuously brought-together bands of film (2, 2') are formed into capsules between two counterrotating forming rolls (3, 3'), a liquid filling material (4) being introduced with the aid of a delivery means (5) into the forming capsule cavity (7) under pressure by way of a filling wedge (6), **characterized in that** the delivery means (5) produces at least one continuous stream of filling material, preferably free from pressure fluctuations, which stream is divided into individual portions by a portioner (8) arranged separately from the delivery means, preferably within the filling wedge, before said portions reach the capsule filling location (9).

2. The method as claimed in claim 1, **characterized in that** the filling material is prepared in the delivery means (5) to form a viscous melt with a temperature of 51° to 200°C.

3. The method as claimed in claim 1 or 2, **characterized in that** the delivery pressure and the delivery distance between the delivery means (5) and the capsule filling location (9) are dimensioned such that, after leaving the delivery means, the filling material reaches the capsule filling location within less than 30 minutes, preferably within less than 10 minutes and still more preferably within less than 3 minutes.

4. The method as claimed in one of claims 1 to 3, **characterized in that** the stream of filling material is divided by the portioner (8) into at least two partial streams, which lead to separate filling locations (9), the division being performed in such a phase-shifted manner that the final capsule filling is achieved at the partial streams one after the other and in each case the second partial stream is released before the first partial stream is interrupted.

5. The method as claimed in one of claims 1 to 4, **characterized in that** the individual portions of the stream of filling material are conducted between the portioner (8) and the capsule filling location (9) over a distance of 0 to at most 50 mm, preferably at most 30 mm.

6. The method as claimed in one of claims 1 to 5, **characterized in that** the filling material is first melted as a mixture of matrix material and active substance and then intermediately stored in the solid, comminuted state before it is melted again and fed to the portioner (8) by the delivery means.

7. The method as claimed in one of claims 1 to 6, **characterized in that** the delivery of the stream of filling material to the portioner (8) is performed with a circulatory displacement delivery means, in particular with a screw pump and preferably with a melt extrusion device.

8. The method as claimed in one of claims 1 to 7, **characterized in that**, when it is dosed at a temperature of 0° to 60°C, preferably at 0° to 50°C, the stream of filling material has a dynamic viscosity of more than 5000 mPa·s or, at a temperature of more than 50°C, a viscosity of at least 10 mPa·s, more preferably of more than 1000 mPa·s, still more preferably of more than 5000 mPa·s.

9. A device for producing soft capsules (1) on the rotary die principle, with two forming rolls (3, 3'), which can be driven counter to each other and between which two bands of film (2, 2') which can be continuously fed in can be formed into capsules, with a filling wedge arranged in the intake region of the forming rolls, for feeding a liquid filling material (4) into the forming capsule cavity (7), and with a delivery means (5) for pressurizing the filling material, **characterized in that**, with the delivery means, at least one continuous stream of filling material can be produced, preferably free from pressure fluctuations, and **in that** the stream of filling material can be divided into individual portions by a portioner (8) arranged separately from the delivery means, preferably within the filling wedge (6), which portions can be fed to the capsule filling location (9).

10. The device as claimed in claim 9, **characterized in that** the delivery means is a circulatory displacement delivery means, in particular a screw pump and preferably a melt extrusion device.

11. The device as claimed in claim 9 or 10, **characterized in that** distributing channels (18) with a maximum length of 50 mm, preferably of 30 mm, are arranged on the surfaces of the filling wedge (6) between the portioner (8) and the capsule filling location (9).

12. The device as claimed in one of claims 9 to 11, **characterized in that** the stream of filling material can be divided at the portioner (8) into at least two partial streams, which can be directed to separate filling locations (9) directly or by way of separate distributing channels, it being possible for the partial streams to be achieved phase-shifted one after the other.

13. The device as claimed in claim 12, **characterized in that**, for feeding the two partial streams at the portioner (8), opening cross sections (18) for each partial stream can be exposed and closed again one after the other on the basis of sinusoidally superposed curves.

14. The device as claimed in one of claims 9 to 13, **characterized in that** the portioner (8) has at least one dosing hollow shaft (21), which is rotatably mounted in the filling wedge (6) and has discharge openings (22), which in at least one rotary angle position coincide with an opening or with a channel in the filling wedge (6).

15. The device as claimed in claim 14, **characterized in that** the dosing hollow shaft (21) is mounted parallel to the axes of rotation (23) of the forming rolls and **in that** each discharge opening (22) can be connected either to a channel leading to the wedge surfaces (24, 24') or to a channel leading to the wedge tip (25) of the filling wedge (6).

16. The device as claimed in one of claims 9 to 13, **characterized in that** the portioner (8) has at least one dosing slide (26, 26'), which is mounted linearly displaceably in the filling wedge (6) and with which feed channels for the filling material can be exposed and closed in a cyclical manner.

17. The device as claimed in claim 16, **characterized in that** the dosing slide is mounted perpendicularly to a plane running through the two axes of rotation of the forming rolls.

18. The device as claimed in claim 16 or 17, **characterized in that** the dosing slide is formed as a dosing tube by way of which the filling material can be fed.

19. The device as claimed in one of claims 9 to 13, **characterized in that** the portioner (8) has at least one valve needle (28), which interacts with a valve seat (29) in the filling wedge.

## Revendications

1. Procédé de fabrication de capsules souples (1) suivant le principe Rotary Die, dans lequel deux bandes de film (2, 2') amenées ensemble en continu sont façonnées en capsules entre deux rouleaux de formage (3, 3') tournant en sens contraire, dans lequel une matière de remplissage liquide (4) est introduite sous pression dans la cavité de capsule en formation (7) à l'aide d'un moyen de transport (5) via un coin de remplissage (6), **caractérisé en ce que** le moyen de transport (5) produit au moins un courant de matière de remplissage continu, de préférence sans variations de pression, qui est partagé en portions individuelles par un doseur (8) disposé séparément du moyen de transport, de préférence à l'intérieur du coin de remplissage, avant que celles-ci n'atteignent le lieu (9) de remplissage des capsules.

2. Procédé selon la revendication 1, **caractérisé en ce que** la matière de remplissage est préparée dans le moyen de transport (5) en un liquide visqueux avec une température de 51° à 200°C.

3. Procédé selon la revendication 1 ou 2, **caractérisé en ce que** la pression de transport et la section de transport entre le moyen de transport (5) et le lieu (9) de remplissage des capsules sont dimensionnées de telle manière que la matière de remplissage, après avoir quitté le moyen de transport, atteigne le lieu de remplissage des capsules en moins de 30 minutes, de préférence en moins de 10 minutes, et de préférence encore en moins de 3 minutes.

4. Procédé selon l'une quelconque des revendications 1 à 3, **caractérisé en ce que** le courant de matière de remplissage est partagé par le doseur (8) en au moins deux courants partiels, qui conduisent à des lieux de remplissage séparés (9), dans lequel le partage est effectué de manière déphasée de telle manière que le remplissage des capsules définitif soit atteint successivement par les courants partiels et que le deuxième courant partiel soit chaque fois libéré avant que le premier courant partiel soit interrompu.

5. Procédé selon l'une quelconque des revendications 1 à 4, **caractérisé en ce que** les portions individuelles du courant de matière de remplissage sont guidées entre le doseur (8) et le lieu (9) de remplissage des capsules sur une distance de 0 à maximum 50 mm, de préférence jusqu'au maximum 30 mm.

6. Procédé selon l'une quelconque des revendications 1 à 5, **caractérisé en ce que** la matière de remplissage est d'abord fondue comme mélange de matière de matrice et de substance active et ensuite stockée provisoirement à l'état solide broyé, avant d'être de nouveau fondue et envoyée par le moyen de transport au doseur (8).

7. Procédé selon l'une quelconque des revendications 1 à 6, **caractérisé en ce que** le transport du courant de matière de remplissage jusqu'au doseur (8) est effectué avec un moyen de transport à déplacement rotatif, en particulier avec une pompe à vis et de préférence avec un dispositif d'extrusion de liquide.

8. Procédé selon l'une quelconque des revendications 1 à 7, **caractérisé en ce que** le courant de matière de remplissage présente lors du dosage, à une température de 0° à 60°C, de préférence de 0° à 50°C, une viscosité dynamique de plus de 5000 mPa·s ou, à une température de plus de 50°C, une viscosité d'au moins 10 mPa·s, de préférence de plus de 1000 mPa·s, et de préférence encore de plus de 5000 mPa·s.

9. Dispositif de fabrication de capsules souples (1) suivant le principe Rotary Die avec deux rouleaux de formage (3, 3') tournant en sens contraire, entre lesquels deux bandes de film (2, 2') amenées ensemble en continu peuvent être façonnées en capsules, avec un coin de remplissage disposé dans la région d'entrée des rouleaux de formage pour amener une matière de remplissage liquide (4) dans la cavité de capsule en formation (7), ainsi qu'avec un moyen de transport (5) pour la mise sous pression de la matière de remplissage, **caractérisé en ce qu'**au moins un courant de matière de remplissage continu, de préférence sans variations de pression, peut être produit avec le moyen de transport et **en ce que** le courant de matière de remplissage peut être partagé en portions individuelles avec un doseur (8) disposé séparément du moyen de transport, de préférence à l'intérieur du coin de remplissage (6), qui peuvent alors être conduites au lieu (9) de remplissage des capsules.

10. Dispositif selon la revendication 9, **caractérisé en ce que** le moyen de transport est un moyen de transport à déplacement rotatif, en particulier une pompe à vis et de préférence un dispositif d'extrusion de liquide.

11. Dispositif selon la revendication 9 ou 10, **caractérisé en ce que** des canaux de distribution (18), d'une longueur maximale de 50 mm, de préférence de 30 mm, sont disposés sur les surfaces du coin de remplissage (6) entre le doseur (8) et le lieu (9) de remplissage des capsules.

12. Dispositif selon l'une quelconque des revendications 9 à 11, **caractérisé en ce que** le courant de matière de remplissage peut être partagé au doseur (8) en au moins deux courants partiels, qui peuvent être conduits à des lieux de remplissage séparés (9) directement ou via des canaux de distribution séparés, dans lequel les courants partiels peuvent être produits successivement de manière déphasée.

13. Dispositif selon la revendication 12, **caractérisé en ce que**, pour la fourniture des deux courants partiels au doseur (8), des sections transversales d'ouvertures (18) pour chaque courant partiel peuvent être libérées et de nouveau fermées successivement selon des courbes se superposant de façon sinusoïdale.

14. Dispositif selon l'une quelconque des revendications 9 à 13, **caractérisé en ce que** le doseur (8) présente au moins un arbre de dosage creux (21), qui est monté de façon rotative dans le coin de remplissage (6) et qui présente des ouvertures de distribution (22) qui correspondent, dans au moins une position angulaire, à une ouverture ou à un canal dans le coin de remplissage (6).

15. Dispositif selon la revendication 14, **caractérisé en ce que** l'arbre creux de dosage (21) est monté parallèlement aux axes de rotation (23) des rouleaux de formage et **en ce que** chaque ouverture de distribution (22) peut être raccordée à un canal menant soit aux faces de coin (24, 24') soit à la pointe de coin (25) du coin de remplissage (6).

16. Dispositif selon l'une quelconque des revendications 9 à 13, **caractérisé en ce que** le doseur (8) présente au moins une vanne de dosage (26, 26'), qui est montée de façon déplaçable linéairement dans le coin de remplissage (6) et avec laquelle les canaux d'amenée pour la matière de remplissage peuvent être ouverts ou fermés en cadence.

17. Dispositif selon la revendication 16, **caractérisé en ce que** la vanne de dosage est montée perpendiculairement à un plan passant par les deux axes de rotation des rouleaux de formage.

18. Dispositif selon la revendication 16 ou la revendication 17, **caractérisé en ce que** la vanne de dosage est constituée par un tube de dosage, à travers lequel la matière de remplissage peut être amenée.

19. Dispositif selon l'une quelconque des revendications 9 à 13, **caractérisé en ce que** le doseur (8) présente au moins un pointeau de soupape (28), qui coopère avec un siège de soupape (29) dans le coin de remplissage.
